# EUROPEAN PATENT APPLICATION

(11) **EP 3 165 924 A1**
(43) Date of publication of application: **10.05.2017**
(21) Application number: 15192849.6
(22) Date of filing: 03.11.2015
(51) Int. Cl.: G01N 33/569

(54) **SCREENING ASSAY FOR INHIBITORS OF HANTAVIRUS**

(71) Applicant: Bernhard-Nocht-Institut für Tropenmedizin, 20359 Hamburg (DE)
(72) Inventor: Günther, Stephan, 22459 Hamburg (DE); Reindl, Sophia, 22297 Hamburg (DE); Fernandez-Garcia, Yaiza, 20359 Hamburg (DE)
(74) Representative: Uexküll & Stolberg

(57) **Abstract**

The invention provides a method for the identification and/or validation of inhibitors of Hantavirus endonuclease and/or drugs for treating or preventing Hantavirus infections, comprising (a) contacting a protein comprising an attenuated Hantavirus endonuclease or a fragment thereof having endonuclease activity with a candidate compound; (b) determining the endonuclease activity of the protein; and (c) comparing the endonuclease activity determined in step (b) with the endonuclease activity of the protein in the absence of the candidate compound; wherein a decreased endonuclease activity of the protein in the presence of the candidate compound indicates that the candidate compound is an inhibitor of Hantavirus endonuclease and/or suitable for treating or preventing Hantavirus infections. Further provided is a protein comprising an attenuated Hantavirus endonuclease or fragment thereof, wherein the endonuclease or fragment thereof comprises a mutation in one or more sequence positions selected from the group consisting of positions corresponding to N167, R35, D40, 143, K44, N50, P96, N98, K124, and K127 of the Andes virus L protein shown in SEQ ID NO:1. Cells expressing the protein and uses of the protein and the cells expressing same are also provided.

SEQUENCE LISTING

<110>  Bernhard-Nocht-Institut für Tropenmedizin

<120>  SCREENING ASSAY FOR INHIBITORS OF HANTAVIRUS

<130>  P 98731

<160>  4

<170>  PatentIn version 3.5

<210>  1
<211>  2153
<212>  PRT
<213>  Andes virus

<400>  1

Met Glu Lys Tyr Arg Glu Ile His Gln Arg Val Arg Asp Leu Ala Pro
1               5                   10                  15

Gly Thr Val Ser Ala Leu Glu Cys Ile Asp Leu Leu Asp Arg Leu Tyr
            20                  25                  30

Ala Val Arg His Asp Leu Val Asp Gln Met Ile Lys His Asp Trp Ser
        35                  40                  45

Asp Asn Lys Asp Val Glu Arg Pro Ile Gly Gln Val Leu Leu Met Ala
    50                  55                  60

Gly Ile Pro Asn Asp Ile Ile Gln Gly Met Glu Lys Lys Ile Ile Pro
65                  70                  75                  80

Asn Ser Pro Ser Gly Gln Val Leu Lys Ser Phe Phe Arg Met Thr Pro
                85                  90                  95

Asp Asn Tyr Lys Ile Thr Gly Asn Leu Ile Glu Phe Ile Glu Val Thr
            100                 105                 110

Val Thr Ala Asp Val Ser Arg Gly Ile Arg Glu Lys Lys Ile Lys Tyr
        115                 120                 125

Glu Gly Gly Leu Gln Phe Val Glu His Leu Leu Glu Thr Glu Ser Arg
    130                 135                 140

Lys Gly Asn Ile Pro Gln Pro Tyr Lys Ile Thr Phe Ser Val Val Ala
145                 150                 155                 160

Val Lys Thr Asp Gly Ser Asn Ile Ser Thr Gln Trp Pro Ser Arg Arg
                165                 170                 175

Asn Asp Gly Val Ile Gln His Met Arg Leu Val Gln Ala Asp Ile Asn
            180                 185                 190

Tyr Val Arg Glu His Leu Ile Lys Leu Asp Glu Arg Ala Ser Leu Glu
        195                 200                 205

Ala Met Phe Asn Leu Lys Phe His Val Ser Gly Pro Lys Leu Arg Tyr
    210                 215                 220

Phe Asn Ile Pro Asp Tyr Arg Pro Gln Gln Leu Cys Glu Pro Arg Ile
225                 230                 235                 240

Asp Asn Leu Ile Gln Tyr Cys Lys Asn Trp Leu Thr Lys Glu His Lys
                245                 250                 255

Phe Val Phe Lys Glu Val Ser Gly Ala Asn Val Ile Gln Ala Phe Glu
            260                 265                 270

Ser His Glu Gln Leu His Leu Gln Lys Tyr Asn Glu Ser Arg Lys Pro
        275                 280                 285

Arg Asn Phe Leu Leu Leu Gln Leu Thr Val Gln Gly Ala Tyr Leu Pro
    290                 295                 300

Ser Thr Ile Ser Ser Asp Gln Cys Asn Thr Arg Ile Gly Cys Leu Glu
305                 310                 315                 320

Ile Ser Lys Asn Gln Pro Glu Thr Pro Val Gln Met Leu Ala Leu Asp
                325                 330                 335

Ile Ser Tyr Lys Tyr Leu Ser Leu Thr Arg Asp Glu Leu Ile Asn Tyr
            340                 345                 350

Tyr Ser Pro Arg Val His Phe Gln Ser Ser Pro Asn Val Lys Glu Pro
        355                 360                 365

Gly Thr Leu Lys Leu Gly Leu Ser Gln Leu Asn Pro Leu Ser Lys Ser
    370                 375                 380

Ile Leu Asp Asn Val Gly Lys His Lys Lys Asp Lys Gly Leu Phe Gly
385                 390                 395                 400

Glu Ile Ile Asp Ser Ile Asn Val Ala Ser Gln Ile Gln Ile Asn Ala
                405                 410                 415

Cys Ala Lys Ile Ile Glu Gln Ile Leu Ser Asn Leu Glu Ile Asn Ile
            420                 425                 430

Gly Glu Ile Asn Ala Ser Met Pro Ser Pro Asn Lys Thr Thr Gly Val
        435                 440                 445

Asp Asp Leu Leu Asn Lys Phe Tyr Asp Asn Glu Leu Gly Lys Tyr Met
    450                 455                 460

Leu Ser Ile Leu Arg Lys Thr Ala Ala Trp His Ile Gly His Leu Val
465                 470                 475                 480

Arg Asp Ile Thr Glu Ser Leu Ile Ala His Ala Gly Leu Arg Arg Ser
                485                 490                 495

Lys Tyr Trp Ser Val His Ala Tyr Asp His Gly Asn Val Ile Leu Phe
            500                 505                 510

Ile Leu Pro Ser Lys Ser Leu Glu Val Val Gly Ser Tyr Ile Arg Tyr
        515                 520                 525

Phe Thr Val Phe Lys Asp Gly Ile Gly Leu Ile Asp Ala Asp Asn Ile
    530                 535                 540

Asp Ser Lys Ala Glu Ile Asp Gly Val Thr Trp Cys Tyr Ser Lys Val
545                 550                 555                 560

Met Ser Ile Asp Leu Asn Arg Leu Leu Ala Leu Asn Ile Ala Phe Glu
                565                 570                 575

Lys Ser Leu Leu Ala Thr Ala Thr Trp Phe Gln Tyr Tyr Thr Glu Asp
            580                 585                 590

Gln Gly His Phe Pro Leu Gln His Ala Leu Arg Ser Ile Phe Ser Phe
        595                 600                 605

His Phe Leu Leu Cys Val Ser Gln Lys Met Lys Leu Cys Ala Ile Phe
    610                 615                 620

Asp Asn Leu Arg Tyr Leu Ile Pro Ser Val Thr Ser Leu Tyr Ser Gly
625                 630                 635                 640

Tyr Glu Leu Leu Ile Glu Lys Phe Phe Glu Arg Pro Phe Lys Ser Ser
                645                 650                 655

Leu Asp Val Tyr Leu Tyr Ser Ile Ile Lys Ser Leu Leu Ile Ser Leu
            660                 665                 670

Ala Gln Asn Asn Lys Val Arg Phe Tyr Ser Arg Val Arg Leu Leu Gly
        675                 680                 685

Leu Thr Val Asp His Ser Thr Val Gly Ala Ser Gly Val Tyr Pro Ser
    690                 695                 700

Leu Met Ser Arg Val Val Tyr Lys His Tyr Arg Ser Leu Ile Ser Glu
705                 710                 715                 720

Ala Thr Thr Cys Phe Phe Leu Phe Glu Lys Gly Leu His Gly Asn Leu
                725                 730                 735

Pro Glu Glu Ala Lys Ile His Leu Glu Thr Ile Glu Trp Ala Arg Lys
            740                 745                 750

Phe Gln Glu Lys Glu Lys Gln Tyr Gly Asp Ile Leu Leu Lys Glu Gly
        755                 760                 765

Tyr Thr Ile Glu Ser Val Ile Asn Gly Glu Val Asp Val Glu Gln Gln
    770                 775                 780

Leu Phe Cys Gln Glu Val Ser Glu Leu Ser Ala Gln Glu Leu Asn Lys
785                 790                 795                 800

Tyr Leu Gln Ala Lys Ser Gln Val Leu Cys Ala Asn Ile Met Asn Lys
                805                 810                 815

His Trp Asp Lys Pro Tyr Phe Ser Gln Thr Arg Asn Ile Ser Leu Lys
            820                 825                 830

Gly Met Ser Gly Ala Leu Gln Glu Asp Gly His Leu Ala Ala Ser Val
        835                 840                 845

Thr Leu Ile Glu Ala Ile Arg Phe Leu Asn Arg Ser Gln Thr Asn Pro
    850                 855                 860

Asn Val Ile Asp Met Tyr Glu Gln Thr Lys Gln Ser Lys Ala Gln Ala
865                 870                 875                 880

Arg Ile Val Arg Lys Tyr Gln Arg Thr Glu Ala Asp Arg Gly Phe Phe
                885                 890                 895

Ile Thr Thr Leu Pro Thr Arg Val Arg Leu Glu Ile Ile Glu Asp Tyr
            900                 905                 910

Phe Asp Ala Ile Ala Lys Val Val Pro Glu Glu Tyr Ile Ser Tyr Gly
        915                 920                 925

Gly Asp Lys Lys Val Leu Asn Ile Gln Asn Ala Leu Glu Lys Ala Leu

    930                 935                 940

Arg Trp Ala Ser Gly Val Ser Glu Ile Thr Thr Ser Thr Gly Lys Ser
945                 950                 955                 960

Ile Lys Phe Lys Arg Lys Leu Met Tyr Val Ser Ala Asp Ala Thr Lys
                965                 970                 975

Trp Ser Pro Gly Asp Asn Ser Ala Lys Phe Arg Arg Phe Thr Gln Ala
            980                 985                 990

Ile Tyr Asp Gly Leu Ser Asp Asn Lys Leu Lys Cys Cys Val Val Asp
        995                 1000                1005

Ala Leu Arg Asn Ile Tyr Glu Thr Glu Phe Phe Met Ser Arg Lys
    1010                1015                1020

Leu His Arg Tyr Ile Asp Ser Met Glu Asn His Ser Asp Ala Val
    1025                1030                1035

Glu Asp Phe Leu Ala Phe Phe Ser Asn Gly Val Ser Ala Asn Val
    1040                1045                1050

Lys Gly Asn Trp Leu Gln Gly Asn Leu Asn Lys Cys Ser Ser Leu
    1055                1060                1065

Phe Gly Ala Ala Val Ser Leu Leu Phe Arg Glu Val Trp Lys Gln
    1070                1075                1080

Leu Phe Pro Glu Leu Glu Cys Phe Phe Glu Phe Ala His His Ser
    1085                1090                1095

Asp Asp Ala Leu Phe Ile Tyr Gly Tyr Leu Glu Pro Glu Asp Asp
    1100                1105                1110

Gly Thr Asp Trp Phe Leu Tyr Val Ser Gln Gln Ile Gln Ala Gly
    1115                1120                1125

Asn Phe His Trp His Ala Ile Asn Gln Glu Met Trp Lys Ser Met
    1130                1135                1140

Phe Asn Leu His Glu His Leu Leu Leu Met Gly Ser Ile Lys Val
    1145                1150                1155

Ser Pro Lys Lys Thr Thr Val Ser Pro Thr Asn Ala Glu Phe Leu
    1160                1165                1170

Ser Thr Phe Phe Glu Gly Cys Ala Val Ser Ile Pro Phe Val Lys
    1175                1180                1185

Ile Leu Leu Gly Ser Leu Ser Asp Leu Pro Gly Leu Gly Phe Phe
    1190                1195                1200

Asp Asp Leu Ala Ala Ala Gln Ser Arg Cys Val Lys Ser Leu Asp
    1205                1210                1215

Leu Gly Ala Cys Pro Gln Leu Ala Gln Leu Ala Ile Val Leu Cys
    1220                1225                1230

Thr Ser Lys Val Glu Arg Leu Tyr Gly Thr Ala Asp Gly Met Val
    1235                1240                1245

Asn Ser Pro Thr Ala Phe Leu Lys Val Asn Lys Ala His Val Pro
    1250                1255                1260

Val Pro Leu Gly Gly Asp Gly Ser Met Ser Ile Met Glu Leu Ala
    1265                1270                1275

Thr Ala Gly Phe Gly Met Ala Asp Lys Asn Ile Leu Lys Asn Ala
    1280                1285                1290

Phe Ile Ser Tyr Lys His Thr Arg Arg Asp Gly Asp Arg Tyr Val
    1295                1300                1305

Leu Gly Leu Phe Lys Phe Leu Met Ser Leu Ser Glu Asp Val Phe
    1310                1315                1320

Gln His Asp Arg Leu Gly Glu Phe Ser Phe Val Gly Lys Val Gln
    1325                1330                1335

Trp Lys Val Phe Thr Pro Lys Ala Glu Phe Glu Phe His Asp Gln
    1340                1345                1350

Phe Ser His Asn Tyr Leu Leu Glu Trp Thr Arg Gln His Pro Val
    1355                1360                1365

Tyr Asp Tyr Ile Ile Pro Arg Asn Arg Asp Asn Leu Leu Val Tyr
    1370                1375                1380

Leu Val Arg Lys Leu Asn Asp Pro Ser Ile Ile Thr Ala Met Thr
    1385                1390                1395

Met Gln Ser Pro Leu Gln Leu Arg Phe Arg Met Gln Ala Lys Gln
    1400                1405                1410

His Met Lys Val Cys Arg Tyr Glu Gly Glu Trp Val Thr Phe Arg
    1415                1420                1425

Glu Val Leu Ala Ala Ala Asp Ser Phe Ala Thr Ser Tyr Gln Pro
    1430                1435                1440

Thr Glu Arg Asp Met Asp Leu Phe Asn Thr Leu Val Ser Cys Thr
    1445                1450                1455

Phe Ser Lys Glu Tyr Ala Trp Lys Asp Phe Leu Asn Glu Val Arg
    1460                1465                1470

Cys Glu Val Leu Thr Thr Arg His Val His Arg Pro Lys Ile Ala
    1475                1480                1485

Arg Thr Phe Thr Val Arg Glu Lys Asp Gln Ala Ile Gln Asn Pro
    1490                1495                1500

Ile Asn Ser Val Ile Gly Tyr Lys Tyr Ala Leu Thr Val Asp Glu
    1505                1510                1515

Val Ser Asp Val Leu Asp Ser Ala Phe Phe Pro Glu Ser Leu Ser
    1520                1525                1530

Ala Asp Leu Gln Val Met Lys Asp Gly Val Tyr Arg Glu Leu Gly
    1535                1540                1545

Leu Asp Ile Ser Ser Pro Glu Val Leu Lys Arg Ile Ala Pro Leu
    1550                1555                1560

Leu Tyr Lys Ala Gly Arg Ser Arg Val Val Ile Val Glu Gly Asn
    1565                1570                1575

Val Glu Gly Thr Ala Glu Ser Ile Cys Ser Tyr Trp Leu Lys Thr
    1580                1585                1590

Met Ser Leu Ile Lys Thr Ile Arg Val Arg Pro Lys Lys Glu Val
    1595                1600                1605

Leu Lys Ala Met Ser Leu Tyr Ser Val Lys Glu Asn Ile Gly Leu
    1610                1615                1620

Gln Asp Asp Ile Ala Ala Thr Arg Leu Cys Ile Glu Ile Trp Arg
    1625                1630                1635

Trp Cys Lys Ala Asn Glu Gln Asp Val Lys Glu Trp Leu Thr Ser
    1640                1645                1650

Leu Tyr Phe Glu Lys Gln Thr Leu Met Asp Trp Val Glu Arg Phe
    1655                1660                1665

Arg Arg Lys Gly Val Val Pro Ile Asp Pro Glu Ile Gln Cys Ile
    1670                1675                1680

Gly Leu Leu Leu Tyr Asp Val Leu Gly Tyr Lys Ser Val Leu Gln
    1685                1690                1695

Met Gln Ala Asn Arg Arg Ala Tyr Ser Gly Lys Gln Tyr Asp Ala
    1700                1705                1710

Tyr Cys Val Gln Thr Tyr Asn Glu Glu Thr Lys Leu Tyr Glu Gly
    1715                1720                1725

Asp Leu Arg Val Thr Phe Asn Phe Gly Leu Asp Cys Ala Arg Leu
    1730                1735                1740

Glu Val Phe Trp Asp Lys Lys Glu Tyr Ile Leu Glu Thr Ser Ile
    1745                1750                1755

Thr Gln Arg His Val Leu Arg Leu Leu Met Glu Glu Val Ser Gln
    1760                1765                1770

Glu Leu Ile Arg Cys Gly Met Arg Phe Lys Thr Glu Gln Val Asn
    1775                1780                1785

Gln Thr Arg Ser Leu Val Leu Phe Lys Thr Glu Ala Gly Phe Glu
    1790                1795                1800

Trp Gly Lys Pro Asn Val Pro Cys Ile Val Tyr Lys His Cys Val
    1805                1810                1815

Leu Arg Thr Gly Leu Arg Thr Lys Gln Pro Ile Asn Lys Glu Phe
    1820                1825                1830

Met Ile Asn Val Gln Ser Asp Gly Phe Arg Ala Ile Ala Gln Met
    1835                1840                1845

Asp Ile Glu Ser Pro Arg Phe Leu Leu Ala His Ala Tyr His Thr
    1850                1855                1860

Leu Arg Asp Ile Arg Tyr Gln Ala Val Gln Ala Val Gly Asn Val
    1865                1870                1875

Trp Phe Lys Thr Glu Gln His Lys Leu Phe Ile Asn Pro Ile Ile

1880                1885                1890

Ser Ser Gly Leu Leu Glu Asn Phe Met Lys Gly Leu Pro Ala Ala
    1895                1900                1905

Ile Pro Pro Ala Ala Tyr Ser Leu Ile Met Asn Lys Ala Lys Ile
    1910                1915                1920

Ser Val Asp Leu Phe Met Phe Asn Glu Leu Leu Ala Leu Ile Asn
    1925                1930                1935

Arg Asn Asn Ile Leu Asn Leu Asp Gly Ile Glu Glu Thr Ser Glu
    1940                1945                1950

Gly Tyr Ser Thr Val Thr Ser Met Ser Ser Lys Gln Trp Ser Glu
    1955                1960                1965

Glu Met Ser Leu Met Ser Asp Asp Asp Ile Asp Asp Met Glu Asp
    1970                1975                1980

Phe Thr Ile Ala Leu Asp Asp Ile Asp Phe Glu Gln Ile Asn Leu
    1985                1990                1995

Glu Glu Asp Ile Gln His Phe Leu Gln Asp Glu Ser Ala Tyr Val
    2000                2005                2010

Gly Asp Leu Leu Ile Gln Thr Glu Asp Ile Glu Val Lys Lys Ile
    2015                2020                2025

Arg Gly Val Thr Arg Val Leu Glu Pro Val Lys Leu Leu Lys Ser
    2030                2035                2040

Trp Val Ser Lys Gly Leu Ala Ile Asp Lys Val Tyr Asn Pro Ile
    2045                2050                2055

Gly Ile Ile Leu Met Ala Arg Tyr Met Ser Lys Thr Tyr Asn Phe
    2060                2065                2070

Ser Ser Thr Pro Leu Ala Leu Leu Asn Pro Tyr Asp Leu Thr Glu
    2075                2080                2085

Leu Glu Ser Val Val Lys Gly Trp Gly Glu Thr Val Asn Asp Arg
    2090                2095                2100

Phe Lys Asp Leu Asp Ile Glu Ala Gln Thr Val Val Lys Glu Lys
    2105                2110                2115

Gly Val Gln Pro Glu Asp Val Leu Pro Asp Ser Leu Phe Ser Phe
    2120                2125                2130

Arg His Val Asp Val Leu Leu Arg Arg Leu Phe Pro Arg Asp Pro
    2135                2140                2145

Val Ser Thr Phe Tyr
    2150

<210> 2
<211> 200
<212> PRT
<213> Andes virus

<400> 2

Met Glu Lys Tyr Arg Glu Ile His Gln Arg Val Arg Asp Leu Ala Pro
1               5                   10                  15

Gly Thr Val Ser Ala Leu Glu Cys Ile Asp Leu Leu Asp Arg Leu Tyr
            20                  25                  30

Ala Val Arg His Asp Leu Val Asp Gln Met Ile Lys His Asp Trp Ser
        35                  40                  45

Asp Asn Lys Asp Val Glu Arg Pro Ile Gly Gln Val Leu Leu Met Ala
    50                  55                  60

Gly Ile Pro Asn Asp Ile Ile Gln Gly Met Glu Lys Lys Ile Ile Pro
65                  70                  75                  80

Asn Ser Pro Ser Gly Gln Val Leu Lys Ser Phe Phe Arg Met Thr Pro
                85                  90                  95

Asp Asn Tyr Lys Ile Thr Gly Asn Leu Ile Glu Phe Ile Glu Val Thr
            100                 105                 110

Val Thr Ala Asp Val Ser Arg Gly Ile Arg Glu Lys Lys Ile Lys Tyr
        115                 120                 125

Glu Gly Gly Leu Gln Phe Val Glu His Leu Leu Glu Thr Glu Ser Arg
    130                 135                 140

Lys Gly Asn Ile Pro Gln Pro Tyr Lys Ile Thr Phe Ser Val Val Ala
145                 150                 155                 160

Val Lys Thr Asp Gly Ser Asn Ile Ser Thr Gln Trp Pro Ser Arg Arg
                165                 170                 175

Asn Asp Gly Val Ile Gln His Met Arg Leu Val Gln Ala Asp Ile Asn
            180                 185                 190

Tyr Val Arg Glu His Leu Ile Lys
        195                 200

<210>  3
<211>  200
<212>  PRT
<213>  Andes virus

<400>  3

Met Glu Lys Tyr Arg Glu Ile His Gln Arg Val Arg Asp Leu Ala Pro
1               5                   10                  15

Gly Thr Val Ser Ala Leu Glu Cys Ile Asp Leu Leu Asp Arg Leu Tyr
            20                  25                  30

Ala Val Arg His Asp Leu Val Asp Gln Met Ile Lys His Asp Trp Ser
        35                  40                  45

Asp Asn Lys Asp Val Glu Arg Pro Ile Gly Gln Val Leu Leu Met Ala
    50                  55                  60

Gly Ile Pro Asn Asp Ile Ile Gln Gly Met Glu Lys Lys Ile Ile Pro
65                  70                  75                  80

Asn Ser Pro Ser Gly Gln Val Leu Lys Ser Phe Phe Arg Met Thr Pro
                85                  90                  95

Asp Asn Tyr Lys Ile Thr Gly Asn Leu Ile Glu Phe Ile Glu Val Thr
            100                 105                 110

Val Thr Ala Asp Val Ser Arg Gly Ile Arg Glu Lys Lys Ile Ala Tyr
        115                 120                 125

Glu Gly Gly Leu Gln Phe Val Glu His Leu Leu Glu Thr Glu Ser Arg
    130                 135                 140

Lys Gly Asn Ile Pro Gln Pro Tyr Lys Ile Thr Phe Ser Val Val Ala
145                 150                 155                 160

Val Lys Thr Asp Gly Ser Asn Ile Ser Thr Gln Trp Pro Ser Arg Arg
                165                 170                 175

Asn Asp Gly Val Ile Gln His Met Arg Leu Val Gln Ala Asp Ile Asn
            180                 185                 190

Tyr Val Arg Glu His Leu Ile Lys
        195                 200

<210>  4
<211>  2153
<212>  PRT
<213>  Andes virus

<400>  4

Met Glu Lys Tyr Arg Glu Ile His Gln Arg Val Arg Asp Leu Ala Pro
1               5                   10                  15

Gly Thr Val Ser Ala Leu Glu Cys Ile Asp Leu Leu Asp Arg Leu Tyr
            20                  25                  30

Ala Val Arg His Asp Leu Val Asp Gln Met Ile Lys His Asp Trp Ser
        35                  40                  45

Asp Asn Lys Asp Val Glu Arg Pro Ile Gly Gln Val Leu Leu Met Ala
    50                  55                  60

Gly Ile Pro Asn Asp Ile Ile Gln Gly Met Glu Lys Lys Ile Ile Pro
65                  70                  75                  80

Asn Ser Pro Ser Gly Gln Val Leu Lys Ser Phe Phe Arg Met Thr Pro
                85                  90                  95

Asp Asn Tyr Lys Ile Thr Gly Asn Leu Ile Glu Phe Ile Glu Val Thr
            100                 105                 110

Val Thr Ala Asp Val Ser Arg Gly Ile Arg Glu Lys Lys Ile Lys Tyr
        115                 120                 125

Glu Gly Gly Leu Gln Phe Val Glu His Leu Leu Glu Thr Glu Ser Arg
    130                 135                 140

Lys Gly Asn Ile Pro Gln Pro Tyr Lys Ile Thr Phe Ser Val Val Ala
145                 150                 155                 160

Val Lys Thr Asp Gly Ser Asn Ile Ser Thr Gln Trp Pro Ser Arg Arg
                165                 170                 175

Asn Asp Gly Val Val Gln His Met Arg Leu Val Gln Ala Asp Ile Asn
            180                 185                 190

Tyr Val Arg Glu His Leu Ile Lys Leu Asp Glu Arg Ala Ser Leu Glu
        195                 200                 205

Ala Met Phe Asn Leu Lys Phe His Val Ser Gly Pro Lys Leu Arg Tyr
    210                 215                 220

Phe Asn Ile Pro Asp Tyr Arg Pro Gln Gln Leu Cys Glu Pro Arg Ile
225                 230                 235                 240

Asp Asn Leu Ile Gln Tyr Cys Lys Asn Trp Leu Thr Lys Glu His Lys
                245                 250                 255

Phe Val Phe Lys Glu Val Ser Gly Ala Asn Val Ile Gln Ala Phe Glu
            260                 265                 270

Ser His Glu Gln Leu His Leu Gln Lys Tyr Asn Glu Ser Arg Lys Pro
        275                 280                 285

Arg Asn Phe Leu Leu Leu Gln Leu Thr Val Gln Gly Ala Tyr Leu Pro
    290                 295                 300

Ser Thr Ile Ser Ser Asp Gln Cys Asn Thr Arg Ile Gly Cys Leu Glu
305                 310                 315                 320

Ile Ser Lys Asn Gln Pro Glu Thr Pro Val Gln Met Leu Ala Leu Asp
                325                 330                 335

Ile Ser Tyr Lys Tyr Leu Ser Leu Thr Arg Asp Glu Leu Ile Asn Tyr
            340                 345                 350

Tyr Ser Pro Arg Val His Phe Gln Ser Ser Pro Asn Val Lys Glu Pro
        355                 360                 365

Gly Thr Leu Lys Leu Gly Leu Ser Gln Leu Asn Pro Leu Ser Lys Ser
    370                 375                 380

Ile Leu Asp Asn Val Gly Lys His Lys Lys Asp Lys Gly Leu Phe Gly
385                 390                 395                 400

Glu Ile Ile Asp Ser Ile Asn Val Ala Ser Gln Ile Gln Ile Asn Ala
                405                 410                 415

Cys Ala Lys Ile Ile Glu Gln Ile Leu Ser Asn Leu Glu Ile Asn Ile
            420                 425                 430

Gly Glu Ile Asn Ala Ser Met Pro Ser Pro Asn Lys Thr Thr Gly Val
        435                 440                 445

Asp Asp Leu Leu Asn Lys Phe Tyr Asp Asn Glu Leu Gly Lys Tyr Met
    450                 455                 460

Leu Ser Ile Leu Arg Lys Thr Ala Ala Trp His Ile Gly His Leu Val
465                 470                 475                 480

Arg Asp Ile Thr Glu Ser Leu Ile Ala His Ala Gly Leu Arg Arg Ser
                485                 490                 495

Lys Tyr Trp Ser Val His Ala Tyr Asp His Gly Asn Val Ile Leu Phe
            500                 505                 510

Ile Leu Pro Ser Lys Ser Leu Glu Val Val Gly Ser Tyr Ile Arg Tyr
        515                 520                 525

Phe Thr Val Phe Lys Asp Gly Ile Gly Leu Ile Asp Ala Asp Asn Ile
    530                 535                 540

Asp Ser Lys Ala Glu Ile Asp Gly Val Thr Trp Cys Tyr Ser Lys Val
545                 550                 555                 560

Met Ser Ile Asp Leu Asn Arg Leu Leu Ala Leu Asn Ile Ala Phe Glu
                565                 570                 575

Lys Ser Leu Leu Ala Thr Ala Thr Trp Phe Gln Tyr Tyr Thr Glu Asp
            580                 585                 590

Gln Gly His Phe Pro Leu Gln His Ala Leu Arg Ser Ile Phe Ser Phe
        595                 600                 605

His Phe Leu Leu Cys Val Ser Gln Lys Met Lys Leu Cys Ala Ile Phe
    610                 615                 620

Asp Asn Leu Arg Tyr Leu Ile Pro Ser Val Thr Ser Leu Tyr Ser Gly
625                 630                 635                 640

Tyr Glu Leu Leu Ile Glu Lys Phe Phe Glu Arg Pro Phe Lys Ser Ser
                645                 650                 655

Leu Asp Val Tyr Leu Tyr Ser Ile Ile Lys Ser Leu Leu Ile Ser Leu
            660                 665                 670

Ala Gln Asn Asn Lys Val Arg Phe Tyr Ser Arg Val Arg Leu Leu Gly
        675                 680                 685

Leu Thr Val Asp His Ser Thr Val Gly Ala Ser Gly Val Tyr Pro Ser
    690                 695                 700

Leu Met Ser Arg Val Val Tyr Lys His Tyr Arg Ser Leu Ile Ser Glu
705                 710                 715                 720

Ala Thr Thr Cys Phe Phe Leu Phe Glu Lys Gly Leu His Gly Asn Leu
                725                 730                 735

Pro Glu Glu Ala Lys Ile His Leu Glu Thr Ile Glu Trp Ala Arg Lys
            740                 745                 750

Phe Gln Glu Lys Glu Lys Gln Tyr Gly Asp Ile Leu Leu Lys Glu Gly
        755                 760                 765

Tyr Thr Ile Glu Ser Val Ile Asn Gly Glu Val Asp Val Glu Gln Gln
    770                 775                 780

Leu Phe Cys Gln Glu Val Ser Glu Leu Ser Ala Gln Glu Leu Asn Lys
785                 790                 795                 800

Tyr Leu Gln Ala Lys Ser Gln Val Leu Cys Ala Asn Ile Met Asn Lys
                805                 810                 815

His Trp Asp Lys Pro Tyr Phe Ser Gln Thr Arg Asn Ile Ser Leu Lys
            820                 825                 830

Gly Met Ser Gly Ala Leu Gln Glu Asp Gly His Leu Ala Ala Ser Val
        835                 840                 845

Thr Leu Ile Glu Ala Ile Arg Phe Leu Asn Arg Ser Gln Thr Asn Pro
    850                 855                 860

Asn Val Ile Asp Met Tyr Glu Gln Thr Lys Gln Ser Lys Ala Gln Ala
865                 870                 875                 880

Arg Ile Val Arg Lys Tyr Gln Arg Thr Glu Ala Asp Arg Gly Phe Phe
                885                 890                 895

Ile Thr Thr Leu Pro Thr Arg Val Arg Leu Glu Ile Ile Glu Asp Tyr
            900                 905                 910

Phe Asp Ala Ile Ala Lys Val Val Pro Glu Glu Tyr Ile Ser Tyr Gly
        915                 920                 925

Gly Asp Lys Lys Val Leu Asn Ile Gln Asn Ala Leu Glu Lys Ala Leu
    930                 935                 940

Arg Trp Ala Ser Gly Val Ser Glu Ile Thr Thr Ser Thr Gly Lys Ser
945                 950                 955                 960

Ile Lys Phe Lys Arg Lys Leu Met Tyr Val Ser Ala Asp Ala Thr Lys

                965                 970                 975

Trp Ser Pro Gly Asp Asn Ser Ala Lys Phe Arg Arg Phe Thr Gln Ala
            980                 985                 990

Ile Tyr Asp Gly Leu Ser Asp Asn Lys Leu Lys Cys Cys Val Val Asp
        995                 1000                1005

Ala Leu Arg Asn Ile Tyr Glu Thr Glu Phe Phe Met Ser Arg Lys
    1010                1015                1020

Leu His Arg Tyr Ile Asp Ser Met Glu Asn His Ser Asp Ala Val
    1025                1030                1035

Glu Asp Phe Leu Ala Phe Phe Ser Asn Gly Val Ser Ala Asn Val
    1040                1045                1050

Lys Gly Asn Trp Leu Gln Gly Asn Leu Asn Lys Cys Ser Ser Leu
    1055                1060                1065

Phe Gly Ala Ala Val Ser Leu Leu Phe Arg Glu Val Trp Lys Gln
    1070                1075                1080

Leu Phe Pro Glu Leu Glu Cys Phe Phe Glu Phe Ala His His Ser
    1085                1090                1095

Asp Asp Ala Leu Phe Ile Tyr Gly Tyr Leu Glu Pro Glu Asp Asp
    1100                1105                1110

Gly Thr Asp Trp Phe Leu Tyr Val Ser Gln Gln Ile Gln Ala Gly
    1115                1120                1125

Asn Phe His Trp His Ala Ile Asn Gln Glu Met Trp Lys Ser Met
    1130                1135                1140

Phe Asn Leu His Glu His Leu Leu Leu Met Gly Ser Ile Lys Val
    1145                1150                1155

Ser Pro Lys Lys Thr Thr Val Ser Pro Thr Asn Ala Glu Phe Leu
    1160                1165                1170

Ser Thr Phe Phe Glu Gly Cys Ala Val Ser Ile Pro Phe Val Lys
    1175                1180                1185

Ile Leu Leu Gly Ser Leu Ser Asp Leu Pro Gly Leu Gly Phe Phe
    1190                1195                1200

Asp Asp Leu Ala Ala Ala Gln Ser Arg Cys Val Lys Ser Leu Asp
    1205                1210                1215

Leu Gly Ala Cys Pro Gln Leu Ala Gln Leu Ala Ile Val Leu Cys
    1220                1225                1230

Thr Ser Lys Val Glu Arg Leu Tyr Gly Thr Ala Asp Gly Met Val
    1235                1240                1245

Asn Ser Pro Thr Ala Phe Leu Lys Val Asn Lys Ala His Val Pro
    1250                1255                1260

Val Pro Leu Gly Gly Asp Gly Ser Met Ser Ile Met Glu Leu Ala
    1265                1270                1275

Thr Ala Gly Phe Gly Met Ala Asp Lys Asn Ile Leu Lys Asn Ala
    1280                1285                1290

Phe Ile Ser Tyr Lys His Thr Arg Arg Asp Gly Asp Arg Tyr Val
    1295                1300                1305

Leu Gly Leu Phe Lys Phe Leu Met Ser Leu Ser Glu Asp Val Phe
    1310                1315                1320

Gln His Asp Arg Leu Gly Glu Phe Ser Phe Val Gly Lys Val Gln
    1325                1330                1335

Trp Lys Val Phe Thr Pro Lys Ala Glu Phe Glu Phe His Asp Gln
    1340                1345                1350

Phe Ser His Asn Tyr Leu Leu Glu Trp Thr Arg Gln His Pro Val
    1355                1360                1365

Tyr Asp Tyr Ile Ile Pro Arg Asn Arg Asp Asn Leu Leu Val Tyr
    1370                1375                1380

Leu Val Arg Lys Leu Asn Asp Pro Ser Ile Ile Thr Ala Met Thr
    1385                1390                1395

Met Gln Ser Pro Leu Gln Leu Arg Phe Arg Met Gln Ala Lys Gln
    1400                1405                1410

His Met Lys Val Cys Arg Tyr Glu Gly Glu Trp Val Thr Phe Arg
    1415                1420                1425

Glu Val Leu Ala Ala Ala Asp Ser Phe Ala Thr Ser Tyr Gln Pro
    1430                1435                1440

Thr Glu Arg Asp Met Asp Leu Phe Asn Thr Leu Val Ser Cys Thr
    1445                1450                1455

Phe Ser Lys Glu Tyr Ala Trp Lys Asp Phe Leu Asn Glu Val Arg
    1460                1465                1470

Cys Glu Val Leu Thr Thr Arg His Val His Arg Pro Lys Ile Ala
    1475                1480                1485

Arg Thr Phe Thr Val Arg Glu Lys Asp Gln Ala Ile Gln Asn Pro
    1490                1495                1500

Ile Asn Ser Val Ile Gly Tyr Lys Tyr Ala Leu Thr Val Asp Glu
    1505                1510                1515

Val Ser Asp Val Leu Asp Ser Ala Phe Phe Pro Glu Ser Leu Ser
    1520                1525                1530

Ala Asp Leu Gln Val Met Lys Asp Gly Val Tyr Arg Glu Leu Gly
    1535                1540                1545

Leu Asp Ile Ser Ser Pro Glu Val Leu Lys Arg Ile Ala Pro Leu
    1550                1555                1560

Leu Tyr Lys Ala Gly Arg Ser Arg Val Val Ile Val Glu Gly Asn
    1565                1570                1575

Val Glu Gly Thr Ala Glu Ser Ile Cys Ser Tyr Trp Leu Lys Thr
    1580                1585                1590

Met Ser Leu Ile Lys Thr Ile Arg Val Arg Pro Lys Lys Glu Val
    1595                1600                1605

Leu Lys Ala Met Ser Leu Tyr Ser Val Lys Glu Asn Ile Gly Leu
    1610                1615                1620

Gln Asp Asp Ile Ala Ala Thr Arg Leu Cys Ile Glu Ile Trp Arg
    1625                1630                1635

Trp Cys Lys Ala Asn Glu Gln Asp Val Lys Glu Trp Leu Thr Ser
    1640                1645                1650

Leu Tyr Phe Glu Lys Gln Thr Leu Met Asp Trp Val Glu Arg Phe
    1655                1660                1665

Arg Arg Lys Gly Val Val Pro Ile Asp Pro Glu Ile Gln Cys Ile
    1670                1675                1680

Gly Leu Leu Leu Tyr Asp Val Leu Gly Tyr Lys Ser Val Leu Gln
    1685                1690                1695

Met Gln Ala Asn Arg Arg Ala Tyr Ser Gly Lys Gln Tyr Asp Ala
    1700                1705                1710

Tyr Cys Val Gln Thr Tyr Asn Glu Glu Thr Lys Leu Tyr Glu Gly
    1715                1720                1725

Asp Leu Arg Val Thr Phe Asn Phe Gly Leu Asp Cys Ala Arg Leu
    1730                1735                1740

Glu Val Phe Trp Asp Lys Lys Glu Tyr Ile Leu Glu Thr Ser Ile
    1745                1750                1755

Thr Gln Arg His Val Leu Arg Leu Leu Met Glu Glu Val Ser Gln
    1760                1765                1770

Glu Leu Ile Arg Cys Gly Met Arg Phe Lys Thr Glu Gln Val Asn
    1775                1780                1785

Gln Thr Arg Ser Leu Val Leu Phe Lys Thr Glu Ala Gly Phe Glu
    1790                1795                1800

Trp Gly Lys Pro Asn Val Pro Cys Ile Val Tyr Lys His Cys Val
    1805                1810                1815

Leu Arg Thr Gly Leu Arg Thr Lys Gln Pro Ile Asn Lys Glu Phe
    1820                1825                1830

Met Ile Asn Val Gln Ser Asp Gly Phe Arg Ala Ile Ala Gln Met
    1835                1840                1845

Asp Ile Glu Ser Pro Arg Phe Leu Leu Ala His Ala Tyr His Thr
    1850                1855                1860

Leu Arg Asp Ile Arg Tyr Gln Ala Val Gln Ala Val Gly Asn Val
    1865                1870                1875

Trp Phe Lys Thr Glu Gln His Lys Leu Phe Ile Asn Pro Ile Ile
    1880                1885                1890

Ser Ser Gly Leu Leu Glu Asn Phe Met Lys Gly Leu Pro Ala Ala
    1895                1900                1905

Ile Pro Pro Ala Ala Tyr Ser Leu Ile Met Asn Lys Ala Lys Ile

1910                1915                1920

Ser Val Asp Leu Phe Met Phe Asn Glu Leu Leu Ala Leu Ile Asn
    1925                1930                1935

Arg Asn Asn Ile Leu Asn Leu Asp Gly Ile Glu Glu Thr Ser Glu
    1940                1945                1950

Gly Tyr Ser Thr Val Thr Ser Met Ser Ser Lys Gln Trp Ser Glu
    1955                1960                1965

Glu Met Ser Leu Met Ser Asp Asp Asp Ile Asp Asp Met Glu Asp
    1970                1975                1980

Phe Thr Ile Ala Leu Asp Asp Ile Asp Phe Glu Gln Ile Asn Leu
    1985                1990                1995

Glu Glu Asp Ile Gln His Phe Leu Gln Asp Glu Ser Ala Tyr Val
    2000                2005                2010

Gly Asp Leu Leu Ile Gln Thr Glu Asp Ile Glu Val Lys Lys Ile
    2015                2020                2025

Arg Gly Val Thr Arg Val Leu Glu Pro Val Lys Leu Leu Lys Ser
    2030                2035                2040

Trp Val Ser Lys Gly Leu Ala Ile Asp Lys Val Tyr Asn Pro Ile
    2045                2050                2055

Gly Ile Ile Leu Met Ala Arg Tyr Met Ser Lys Thr Tyr Asn Phe
    2060                2065                2070

Ser Ser Thr Pro Leu Ala Leu Leu Asn Pro Tyr Asp Leu Thr Glu
    2075                2080                2085

Leu Glu Ser Val Val Lys Gly Trp Gly Glu Thr Val Asn Asp Arg
    2090                2095                2100

Phe Lys Asp Leu Asp Ile Glu Ala Gln Thr Val Val Lys Glu Lys
    2105                2110                2115

Gly Val Gln Pro Glu Asp Val Leu Pro Asp Ser Leu Phe Ser Phe
    2120                2125                2130

Arg His Val Asp Val Leu Leu Arg Arg Leu Phe Pro Arg Asp Pro
    2135                2140                2145

Val Ser  Thr Phe Tyr
    2150

## Description

The invention provides a method for the identification and/or validation of inhibitors of Hantavirus endonuclease and/or drugs for treating or preventing Hantavirus infections, comprising (a) contacting a protein comprising an attenuated Hantavirus endonuclease or a fragment thereof having endonuclease activity with a candidate compound; (b) determining the endonuclease activity of the protein; and (c) comparing the endonuclease activity determined in step (b) with the endonuclease activity of the protein in the absence of the candidate compound; wherein a decreased endonuclease activity of the protein in the presence of the candidate compound indicates that the candidate compound is an inhibitor of Hantavirus endonuclease and/or suitable for treating or preventing Hantavirus infections. Further provided is a protein comprising an attenuated Hantavirus endonuclease or fragment thereof, wherein the endonuclease or fragment thereof comprises a mutation in one or more sequence positions selected from the group consisting of positions corresponding to N167, R35, D40, I43, K44, N50, P96, N98, K124, and K127 of the Andes virus L protein shown in SEQ ID NO:1. Cells expressing the protein and uses of the protein and the cells expressing same are also provided.

### BACKGROUND

Hantaviruses are a major class of human pathogens. They are distributed worldwide, can cause severe diseases in humans and are therefore a major public health threat (Macneil et al., Virus Res, 2011, 162(I-2):138-47; Jonsson et al., Clin Microbiol Rev, 2010, 23(2):412-41; Manigold et al., Swiss Med Wkly, 2014, 144:wl3937). Hantaviruses belong to the family *Bunyaviridae* and use rodents as their primary host (Schountz et al., Viruses, 2014, 6(3):1317-35). For many zoonotic viruses, human infections require the contact to infected rodents or their excrements, but for some Hantaviruses person-to-person transmission has been reported (Wells et al., Emerg Infect Dis, 1997, 3(3):361-5). One of these Hantaviruses is the Andes virus (ANDV), which can cause a respiratory disease called Hantavirus cardiopulmonary syndrome (HCPS) that is associated with a case fatality of up to 40%. ANDV is endemic in Argentina and Chile and its main reservoir host is the long-tailed pygmy rice rat (*Oligoryzomys longicaudatus*) (Medina et al., J Virol, 2009, 83(6):2446-59). Related HCPS-causing Hantaviruses are found throughout the Americas.

The Hantavirus genome consists of three differently sized antisense RNA segments, the L (large), M (medium), and S (small) segment (Hepojoki et al., J Gen Virol, 2012, 93(Pt 8):1631-44). The L segment encodes the 250-kDa L protein, which contains an RNA-dependent RNA polymerase (RdRp) domain in the center, as shown by homologies to polymerase proteins of other RNA viruses (Kukkonen et al., Arch Virol, 2005, 150(3):533-56). This protein - like its homolog partners from other viruses - most likely possesses several additional enzymatic functions involved in RNA transcription and replication. The M segment contains the gene for the glycoprotein precursor (GPC), which is cleaved posttranslationally into the envelope proteins Gn and Gc. The S segment encodes the nucleoprotein (N), which encapsidates the viral genome.

For many Hantaviruses a second gene product from the S-segment was identified, the non-structural protein NSs. It is a very small protein with less than 8 kDa, which seems to play a role in modulating the immune response of the host cell (Jaaskelainen et al, J Med Virol, 2007, 79(10):1527-36; Vera-Otarola et al, J Virol, 2012, 86(4):2176-87; Cimica et al., MBio, 2014, 5(1)).

Around 14 heterologous nucleotides can be found at the 5'-end of Hantavirus mRNAs, suggesting the virus uses a mechanism called 'cap-snatching' to initiate transcription of its mRNAs (Cheng et al., J Virol, 2012, 86[18]:10173-85; Garcin et al., J Virol, 1995, 69(9):5754-62). This viral strategy of stealing a 5'-cap from the host cell mRNAs was thoroughly investigated on the influenza A virus (IAV; family *Orthomyxoviridae*): a viral protein binds to the 5'-cap of a cellular mRNA, which is then cleaved several nucleotides downstream by the endonuclease activity of the viral polymerase protein. The cleaved 5'-capped RNA segment is used as a primer to initiate transcription of the viral mRNA by the host transcription machinery (Plotch et al., Cell, 1981, 23(3):847-58; Dias et al., Nature, 2009, 458(7240):914-8). This endonuclease resides in the very N-terminus of the L protein, as shown for La Crosse virus (LACV; genus Orthobunyavirus, family *Bunyaviridae*)*,* Lassa virus and lymphocytic choriomeningitis virus (family *Arenaviridae*) (Reguera et al., PLoS Pathog, 2010, 6(9):el001101; Morin et al., PLoS Pathog, 2010, 6(9):el001038, Wallat et al., PLoS One, 2014, 9(2):e87577). An endonuclease motif is also found in the N-terminus of Hantavirus L protein, suggesting a functional homology to other L proteins in this region. The cap-snatching mechanism is an essential virus specific process, which makes it an attractive target for novel antiviral therapy.

However, research on ANDV and other Hantaviruses is extremely challenging since due to the low-expression phenotype of the L protein, cells transfected with an L protein expression plasmid do not produce a detectable amount of protein. It was recently shown that the endonuclease of ANDV L protein down-regulates the level of its own mRNA as well as the level of heterologous mRNAs (Heinemann et al., J Virol, 2013, 87(12):6975-85). The authors also concluded that all generated endonuclease mutants which were expressible *in vitro* were endonuclease-negative.

There is, thus, a need in the art to identify suitable constructs which can be expressed in high quantities for use in drug screening assays. The Hantavirus endonuclease should exhibit a cap-snatching endonuclease activity that can be blocked by known inhibitors, such as DPBA, to be able to use the endonuclease for drug screening assays.

### DESCRIPTION

The present invention surprisingly identified Hantavirus endonuclease mutants which show an attenuated enzymatic activity and are hence expressible in recombinant expression systems. These mutants are highly suitable in drug screening assays. The invention, thus, provides in a first aspect a method for the identification and/or validation of inhibitors of Hantavirus endonuclease and/or drugs for treating or preventing Hantavirus infections, comprising (a) contacting a protein comprising an attenuated Hantavirus endonuclease or a fragment thereof having endonuclease activity with a candidate compound; (b) determining the endonuclease activity of the protein; and (c) comparing the endonuclease activity determined in step (b) with the endonuclease activity of the protein in the absence of the candidate compound. A decreased endonuclease activity of the protein in the presence of the candidate compound indicates that the candidate compound is an inhibitor of Hantavirus endonuclease and/or is suitable for treating or preventing Hantavirus infections.

The method comprises in step (a) the contacting of a protein comprising an attenuated Hantavirus endonuclease or a fragment thereof having endonuclease activity with a candidate compound. The step of contacting can be performed, e.g., by mixing the endonuclease-containing protein with the candidate compound, either by adding the protein to the candidate compound or by adding the candidate compound to the protein. The order by which the two components are mixed with each other will generally not be critical for the method of the invention.

The protein used in the present method comprises an attenuated Hantavirus endonuclease or an enzymatically active fragment derived from such Hantavirus endonuclease. As used herein, the term "endonuclease" refers to any protein or polypeptide which shows endonuclease activity, i.e. an enzymatic activity which results in cleavage of the phosphodiester bonds within a polynucleotide chain. The term comprises both wildtype endonucleases, as they occur, e.g., at the N-terminus of the Hantavirus L protein, and variants of naturally occurring endonucleases, in particular mutant endonucleases which comprise one or more mutations relative to the wildtype endonuclease. The endonuclease used in the method of the invention may be comprised in a larger protein, such as in a viral protein, e.g. a Hantavirus L protein, or it may be present as a discrete protein without sequences derived from non-endonuclease proteins. Preferably, the endonuclease which is used in the method of the present invention is a recombinant protein, i.e. a protein produced by using methods of genetic engineering.

For example, the endonuclease protein or fragment used in the method of the present invention can be a protein that has been expressed in a heterologous expression system, e.g. in prokaryotic or eukaryotic expression system. Prokaryotic expression systems include those which are based on bacterial cells such as *Escherichia coli,* e.g. strain BL21. Eukaryotic expression systems include those which are based on yeast or mammalian cells. In one embodiment, the heterologous expression system is a prokaryotic expression system. In a particularly preferred embodiment, the prokaryotic expression system is based on *Escherichia coli* cells. *Escherichia coli* strains like BL21 are particularly preferred.

In a particularly preferred embodiment of the invention, the endonuclease or endonuclease fragment is derived from the ANDV L protein set forth in SEQ ID NO:1. In another preferred embodiment of the invention, the endonuclease or endonuclease fragment is derived from the ANDV L protein set forth in SEQ ID NO:4. In relation to the endonuclease region of the L protein shown in SEQ ID NO:1, the corresponding region of the L protein depicted in SEQ ID NO:4 comprises the amino acid valine in position 181.

As outlined above, the naturally occurring Hantavirus endonuclease is located in the N-terminal part of the Hantavirus L protein. As shown in the below examples, the enzymatic activity of the ANDV endonuclease resides within the 200 N-terminal amino acids of the ANDV L protein. However, it is to be expected that other fragments within the first 200 amino acids would be enzymatically active as well. Accordingly, in one embodiment, the endonuclease for use in the method of the invention comprises or essentially corresponds to amino acids 1-160, 1-165, 1-170, 1-175, 1-180, 1-185, 1-190, 1-195, 10-160, 10-165, 10-170, 10-175, 10-180, 10-185, 10-190, 10-195, 15-160, 15-165, 15-170, 15-175, 15-180, 15-185, 15-190, 15-195, 20-160, 20-165, 20-170, 20-175, 20-180, 20-185, 20-190, 20-195, 25-160, 25-165, 25-170, 25-175, 25-180, 25-185, 25-190, 25-195, 30-160, 30-165, 30-170, 30-175, 30-180, 30-185, 30-190, 30-195, 35-160, 35-165, 35-170, 35-175, 35-180, 35-185, 35-190, or amino acids 35-195.

It is particularly preferred that the endonuclease for use in the method of the invention comprises or consists of at least amino acids 1-180, 1-190 or 1-200 of the sequence of SEQ ID NO:1 or 4.

The endonuclease or endonuclease fragment used in the methods of the invention is attenuated in terms of its endonuclease activity relative to the wild-type enzyme. As used herein, an attenuated endonuclease or endonuclease fragment is one that is less toxic for the cell expressing the endonuclease. As explained above the expression of Hantavirus endonucleases has so far not been possible. Thus, an "attenuated" Hantavirus endonuclease or endonuclease fragment is one which can be expressed in a heterologous expression system.

Methods for the detection of heterologous expression are well known in the art. The expression of endonuclease can be measured on the mRNA level or the protein level. Preferably, the expression of the endonuclease is measured on the protein level. Methods for the determination of protein levels are well known in the art and include, e.g. Western Blot and other methods, such as other methods which use antibodies specific for the expressed protein, e.g. as histological methods. Heterologously expressed endonuclease may be labeled with tags that can either be detected themselves (His-tag, Myc-tag and the like) or serve as markers (fluorescent labels and the like). In a preferred embodiment, the endonuclease or fragment thereof is regarded as an "attenuated" endonuclease when it can be expressed in a heterologous expression system as detected by means of a Western Blot.

The attenuation of the endonuclease or fragment thereof is preferably achieved by the introduction of one or more mutations into the sequence of the wildtype endonuclease, e.g. the sequence depicted in SEQ ID NO:2. In particular, in a preferred embodiment, the endonuclease or fragment thereof comprises one or more mutations in relation to the wildtype ANDV endonuclease, e.g. the sequence set forth in SEQ ID NO:2.

For example, the attenuated Hantavirus endonuclease can comprise a mutation in an amino acid involved in the stabilization of the tertiary structure of the endonuclease. While the secondary structure of a protein is a measure of the local three-dimensional structures of a protein, which are described as e.g. alpha helices and beta sheets, the tertiary structure describes the relative orientation of these secondary structures to each other in three dimensional space, e.g. the geometric shape of a protein which is a result of the interaction between amino acids of the same or different secondary structural elements.

The amino acids involved in the stabilization of the tertiary structure of the Hantavirus endonuclease form e.g. hydrophobic interactions, salt bridges or hydrogen bonds with other amino acids of the protein, whereby different elements of the secondary structure are connected. Thus, the hydrophobic interactions, salt bridges or hydrogen bonds are n not formed between amino acids that are immediately adjacent in the primary sequence. For example, the amino acids involved in the stabilization of the tertiary structure form (i.e. participate in) hydrophobic interactions, salt bridges or hydrogen bonds between two or more α-helices, two or more β-strands, or one or more α-helices and one or more β-strands. Preferably, these hydrophobic interactions, salt bridges or hydrogen bonds are formed with side chains of the amino acids involved in the stabilization of the tertiary structure. It is particularly preferred that the amino acids involved in the stabilization of the tertiary structure are involved in hydrophobic interactions or hydrogen bonds between two or more α-helices, two or more β-strands, or one or more α-helices and one or more β-strands.

In other words, the amino acids involved in the stabilization of the tertiary structure are amino acids which are required to maintain the tertiary structure of the wildtype Hantavirus endonuclease, such as the endonuclease shown in SEQ ID NO:2. Suitable methods for determining whether an amino acid is required to maintain the tertiary structure are known in the art and include e.g. the crystallization and structure determination of respective mutant proteins as described elsewhere herein. In a particularly preferred embodiment, the amino acid is involved in an interaction of amino acids from at least two different secondary structural elements, such as two or more α-helices, two or more β-strands, or one or more α-helices and one or more β-strands.

The endonuclease secondary structures discussed herein have been numbered based on the crystal structure in accordance with the standard nomenclature that is in the art. The crystal structure of the Hantavirus endonuclease has been solved based on the ANDV L¹⁻²⁰⁰ K127A protein mutant which comprises the 200 N-terminal amino acids of the ANDV L-protein (SEQ ID NO:1) and a mutation to alanine in position K127. The sequence of the ANDV L¹⁻²⁰⁰ K127A endonuclease is shown in SEQ ID NO:3. This sequence was used for the crystallization and structure determination experiments described in below Example 2. It was found that the Hantavirus endonuclease comprises eight α-helices and three β-strands.

Specifically, within the ANDV L¹⁻²⁰⁰ K127A endonuclease shown in SEQ ID NO:3, helix α1 consists of amino acid residues 1-13, helix α2 consists of amino acid residues 23-46, helix α3 consists of amino acid residues 57-64, helix α4 consists of amino acid residues 68-75, helix α5 consists of amino acid residues 88-93, helix α6 consists of amino acid residues 117-145, helix α7 consists of amino acid residues 167-171, and helix α8 consists of amino acid residues 176-199. Strand β1 consists of amino acid residues 98-102, strand β2 consists of amino acid residues 105-114, and strand β3 consists of amino acid residues 153-162.

It is particularly preferred that the attenuated endonuclease comprises a mutation in an amino acid that (i) is involved in positioning the loop that contains the PD motif of the active site and/or forms a hydrogen bond in or with the loop containing the PD motif of the active site (but is not comprised in the active site as defined elsewhere herein), (ii) forms hydrophobic interactions between the end of helix α2 and hydrophobic side chains both from the β-strands 2 and 3 and from helix α7, or (iii) forms salt bridges between the small helix α7 and main chain atoms of strand β3. Preferably, the mutation is in an amino acid that is involved in hydrogen bonds with main chain atoms of strand β3.

More preferably, the attenuated endonuclease or fragment thereof comprises a mutation in one or more positions that correspond to positions R35, D40, I43, K44, N50, P96, N98, K124, K127, and N167 of the Andes virus L protein shown in SEQ ID NO:1. Even more preferably the mutation is located in one or more positions corresponding to D40, I43, K44, N98, and N167 of the Andes virus L protein shown in SEQ ID NO:1. As SEQ ID NO:2 (the endonuclease region) is identical to the 200 N-terminal amino acids of SEQ ID NO:1 (the entire L protein), the comparison may also be made with in relation to SEQ ID NO:2 instead of SEQ ID NO:1.

An amino acid position "corresponding to" an amino acid position in a particular sequence, such as SEQ ID NO:2, is a position that is aligned with the latter amino acid position when the sequence in which it is comprised is aligned with the particular sequence (such as SEQ ID NO:2). Methods for sequence alignments are known and standard in the art.

The mutation(s) according to the invention are preferably mutations which do not render the endonuclease inactive but reduce the endonuclease activity of the protein. In other words, the mutation(s) are attenuating mutations, i.e. mutations which attenuate the endonuclease activity of the endonuclease or fragment thereof.

It has been surprisingly found that mutations in these above recited positions result in mutants having endonuclease activity which can, nevertheless, be expressed in a heterologous expression system, such as *E. coli* (compare experimental results described elsewhere herein). R35, N50, P96, K124, and K127 mutants have been found to show a medium endonuclease activity, while D40, I43, K44, and N98 mutants have been found to show a high endonuclease activity, and N167 mutants have been found to show a very high endonuclease activity. Accordingly, in a particularly preferred embodiment, the endonuclease or fragment thereof comprises a mutation in the position corresponding to N167 of SEQ ID NO:1.

In another preferred embodiment, the one or more mutations which result in an attenuated Hantavirus endonuclease or fragment thereof are located outside the active site of the endonuclease. It has been found that mutations concerning residues of the active site lead to a dramatically reduced or no endonuclease activity. Accordingly, endonucleases comprising mutations outside of the active site are better suited for the methods described herein.

The active site is the region of the endonuclease where the substrate molecules, i.e. the nucleic acid to be cleaved as well as two manganese ions, bind and undergo a chemical reaction. The active site of the endonuclease comprises amino acid positions H36, P96, D97, E110, K124, and K127 of SEQ ID NO:2. Outside of the active site means that none of the amino acids corresponding to any of the above positions is mutated. In other words, the attenuated Hantavirus endonuclease or fragment thereof preferably does not comprise a mutation in a position corresponding to any of amino acids H36, P96, D97, E110, K124, and K127 of SEQ ID NO:1.

The one or more mutations which are responsible for the attenuation can be a deletion or substitution of amino acids. It is preferred that the mutation is a substitution, more preferably a non-conservative substitution, i.e. a substitution of one or more amino acid residues by an amino acid of a different polarity, which does not act as a functional equivalent. Preferably, the amino acid residue used as a substitute is selected from a different group of amino acids as the amino acid residue to be substituted. Groups of amino acids are, for example, hydrophobic amino acids, polar amino acids, amino acids having the same charge. For example, non-polar amino acids include glycine, valine, alanine, isoleucine, leucine, methionine, proline, phenylalanine, and tryptophan. Uncharged polar amino acids comprise serine, threonine, glutamine, asparagine, tyrosine and cysteine. Charged polar (basic) amino acids comprise histidine, arginine and lysine. Charged polar (acidic) amino acids comprise aspartic acid and glutamic acid. Thus, for example, an uncharged polar amino acid, such as asparagine (N), is preferably substituted with a non-polar amino acid, such as alanine (A). A charged polar (basic) amino acid, such as lysine, may e.g. is preferably substituted with a non-polar amino acid, such as alanine (A).

For example, it is preferred that the endonuclease or fragment thereof comprises a D40E, I43A, K44A, N50A, P96A, N98A, K124A, K127A, or N167A mutation at the position corresponding to D40, I43, K44, N50, P96, N98, K124, K127 and N167, respectively, of the protein shown in SEQ ID NO: 2. More preferably, the endonuclease or fragment thereof comprises a D40E, I43A, K44A, N98A, or N167A mutation at the position corresponding to D40, I43, K44, N98, and N167, respectively, of the protein shown in SEQ ID NO: 2. In a particularly preferred embodiment, the endonuclease or fragment thereof comprises an alanine at the sequence position corresponding to N167 of the protein of SEQ ID NO: 2.

Despite of the one or more mutations which result in the attenuation of the endonuclease or the fragment thereof, the protein to be used in the method of the invention will exhibit a high degree of sequence identity to the endonuclease shown in SEQ ID NO:2. It is preferred that the endonuclease or the fragment thereof which is used in the method will have a sequence identity of at least 75%, 80%, 85%, 90%, 91%, 92%, 93%, 94%, 95%, 96%, 97%, 98% or 99% to the amino acid sequence of the wildtype endonuclease, preferably the sequence of SEQ ID NO:2, if these sequences are optimally aligned, for example, by the programs GAP or BESTFIT using default gap.

In other words, the protein used in the method of the invention can show from 75% to 99%, preferably from 85% to 99%, or more preferably from 95% to 99% sequence identity to the wildtype endonuclease from which it was derived, e.g. the sequence of SEQ ID NO:2. A sequence identity of 90% means that 90% of the amino acids of an analyzed amino acid sequence stretch are identical to the sequence of the reference amino acid sequence. Preferably, the sequence identity is determined over a stretch of at least 80 amino acids, at least 90 amino acids, at least 100 amino acids, at least 120 amino acids, at least 130 amino acids, at least 140 amino acids, at least 150 amino acids, at least 160 amino acids, at least 170 amino acids, at least 180 amino acids, at least 190 amino acids, or at least 200 amino acids. Preferably, the sequence identity is from 95% to 99% over a stretch of at least 150 amino acids, more preferably over a stretch of at least 180 amino acids, and even more preferably over a stretch of at least 180 amino acids.

The endonuclease or fragment thereof which is used in the method of the invention has preferably a single point mutation in relation to the wildtype endonuclease sequence, e.g. the sequence depicted in SEQ ID NO: 2, over a stretch of 180 amino acids, preferably 200 amino acids. Methods and computer programs for determining amino acid sequence homologies are well known in the art.

As used herein, a fragment of an endonuclease refers to a part of an endonuclease, e.g. of the endonuclease shown in SEQ ID NO:2. The fragment is, thus, a polypeptide identical to a continuous stretch of amino acids in the endonuclease. Hence, the fragment may have a length of at least 50%, at least 55%, at least 60%, at least 65%, at least 70%, at least 75%, at least 80%, at least 85%, at least 90% or at least 95% of the length of the endonuclease shown in SEQ ID NO:2. Preferably, the fragment has a length of at least 75% of the full length of the endonuclease from which it is derived. In other words, the fragment has a length of between 50%-95%, 55%-95%, 60%-95%, 65%-90%, 70%-90%, 75%-90%, 80%-90%, preferably 85% of the full length of the endonuclease from which it is derived.

The attenuated endonuclease or endonuclease fragment, e.g. the endonuclease or endonuclease fragment comprising one or more of the mutations in positions corresponding to D40, I43, K44, N98, and N167 of SEQ ID NO:1 or 2, has an attenuated but detectable endonuclease activity. A protein is considered to have endonuclease activity when it is able to cleave the phosphodiester bond within a polynucleotide chain. Methods for determining endonuclease activity of a protein are well known in the art and described elsewhere herein.

According to the step (a) of the method, said attenuated Hantavirus endonuclease or fragment thereof is contacted with a candidate inhibitor compound. The candidate compound may be any compound and also includes chemical complexes of several compounds. The candidate compound can, thus, e.g. be a small molecule, such as a molecule comprised in a small molecule library that can be used in a medium- or throughput screening. However, the candidate compound may also be a biological molecule, such as a protein or peptide, or a nucleic acid. For example, the candidate compound can be an antibody or a fragment thereof.

According to the invention, steps (a) and (b) can be in the presence of manganese, such as manganese chloride. It is particularly preferred that manganese, such as manganese chloride, is present in a concentration of from 0.1 to 10 mM, preferably 0.5 to 5 mM, more preferably 1 to 3 mM. The skilled artisan is able to identify the optimal manganese concentration by routine testing.

The method comprises a step (b) wherein the endonuclease activity of the protein is determined. Methods for determining the endonuclease activity are well known in the art. Any method for assessing the endonuclease activity known in the art is suitable in the context of the invention.

For example, the endonuclease activity of the protein can be determined in step (b) by incubating the endonuclease with a labeled DNA or RNA oligonucleotide substrate and subsequently determining the integrity and/or the decomposition of the substrate. Suitable labels are known in the art. For example, the oligonucleotide may be labeled with a 6-carboxy-fluorescein (FAM) fluorophore at the 5'-end and a minor groove binding non-fluorescent quencher (MGBNFQ, Applied Biosystems) at the 3'-end. When excited, the MGBNFQ quenches the fluorescence of FAM via FRET, until the substrate is cleaved and the quenching reaction impeded. Alternatively, the oligonucleotide may be labeled with a radioactive label, such as the isotope ³²P of phosphorous. The endonuclease activity can then e.g. be assessed by analyzing the integrity of the substrate on polyacrylamide gel electrophoresis followed by phosphor screen autoradiography.

The Hantavirus endonuclease is known to cleave any single stranded or double stranded RNA and ssDNA *in vitro.* The affinity of the enzyme follows the order ssRNA>dsRNA>ssDNA>dsDNA. Accordingly, the substrate used in step (b) of the method of the invention may be DNA or RNA. The substrate will have a length of at least 15 nucleotides, at least 20 nucleotides, at least 50 nucleotides, at least 100 nucleotides, at least 150 nucleotides, at least 200 nucleotides, at least 250 nucleotides, or longer. Preferably the substrate will be ssRNA or dsRNA.

Alternatively, the endonuclease or fragment thereof can be crystallized and the influence of the candidate compound on the endonuclease activity tested by analyzing a crystal comprising the protein and the candidate compound. The candidate compound may be crystallized together with the protein or may be soaked into the protein crystal after crystallization of the protein (e.g. as described elsewhere herein). The crystal is then subjected to X-ray diffraction analysis to analyze the binding of the candidate compound. Due to the X ray analysis described herein, it is known which intramolecular protein interactions are particularly important for the activity of the endonuclease. Accordingly, the skilled artisan would be able to determine based on the information disclosed herein and the new crystal structure comprising the candidate compound to determine whether the compound can be considered an inhibitor of the endonuclease activity.

The method further comprises a step (c) wherein the endonuclease activity of step (b), i.e. the endonuclease activity determined in step (b), is compared with the endonuclease activity of the protein in the absence of the candidate compound. Preferably, the endonuclease activity in the absence and the presence of the candidate compound is determined under substantially the same reaction conditions, i.e. under reaction conditions which differ only in the presence of the candidate compound. Thus, the same test will be used for assessing the endonuclease activity in the presence and absence of the candidate compound. Of course, the two measurements can be performed at different times and in any order.

According to the invention, a decreased endonuclease activity of the protein in the presence of the candidate compound relative to the corresponding activity of the protein in the absence of said candidate compound indicates that the candidate compound is an inhibitor of Hantavirus endonuclease and/or suitable for treating or preventing Hantavirus infections.

In a further embodiment, the method comprises an additional step (d), wherein the endonuclease activity determined in step (b) is compared with the endonuclease activity of the protein in the presence of a known inhibitor of the endonuclease. Known inhibitors of Hantavirus endonuclease include, e.g. DPBA (2,4-dioxo-4-phenylbutanoic acid).

According to the invention, a decreased endonuclease activity of the protein in the presence of the candidate compound in comparison to the presence of the known inhibitor of the endonuclease indicates that the candidate compound is a particularly potent and preferred inhibitor of Hantavirus endonuclease and/or particularly suitable for treating or preventing Hantavirus infections.

The method of the invention can advantageously be carried out in a high- or medium-throughput format because the attenuated endonuclease or fragment thereof can be expressed *in vitro* in heterologous expression systems in an amount that is sufficiently high for such assays. Thus, the method is preferably a medium- or high-throughput method, such as a medium- or high-throughput assay.

The method according to the invention is suitable for identifying compounds which are useful for treating and preventing Hantavirus infections. As used herein the Hantavirus infection is preferably an infection with a virus selected from the Hantaviruses mentioned herein below. Methods for the diagnosis of a Hantavirus infection are well known in the art and include, e.g. a confirmation of the infection by detection of viral DNA in a patient's sample. In a particularly preferred embodiment, the method of the invention is for the identification and/or validation of inhibitors of Andes virus endonuclease and/or drugs for treating or preventing Andes virus infections.

The Hantavirus can be a virus selected from the group consisting of Andes virus, Amur virus, Asama virus, Azagny virus, Bayou virus, Black Creek Canal virus, Bloodland Lake virus, Blue River virus, Cano Delgadito virus, Calabazo virus, Carrizal virus, Catacamas virus, Choclo virus, Dobrava-Belgrade virus, El Moro Canyon virus, Gou virus, Hantaan River virus, Huitzilac virus, Imjin virus, Isla Vista virus, Khabarovsk virus, Laguna Negra virus, Limestone Canyon virus, Magboi virus, Maripa virus, Monongahela virus, Montano virus, Mouyassue virus, Muleshoe virus, Muju virus, New York virus, Nova virus, Oran virus, Oxbow virus, Playa de Oro virus, Prospect Hill virus, Puumala virus, Rockport virus, Rio Mamore virus, Rio Segundo virus, Sangassou virus, Saaremaa virus, Seoul virus, Serang virus, Sin Nombre virus, Soochong virus, Tanganya virus, Thailand virus, Thottapalayam virus, Topografov virus, Tula virus, or Xuan Son virus. It is particularly preferred that the Hantavirus is from Andes virus (ANDV), which means that endonuclease used in the method of the invention is an endonuclease of ANDV or a fragment thereof.

In a further aspect, the invention relates to a protein comprising the attenuated Hantavirus endonuclease described herein, wherein the endonuclease comprises a mutation in one or more positions corresponding to R35, D40, I43, K44, N50, P96, N98, K124, K127, and N167 of the Andes virus L protein shown in SEQ ID NO:1 or 2, preferably positions corresponding to D40, I43, K44, N50, N98, and N167 of the Andes virus L protein shown in SEQ ID NO:1, most preferably position corresponding to N167 of the Andes virus L protein shown in SEQ ID NO:1. Any of the mutations described elsewhere herein in regard to the method can be included in the protein of the invention. The protein preferably is a recombinant protein.

In yet another aspect the invention relates to a cell, preferably a bacterial or yeast cell, expressing a protein as described herein.

The invention also pertains to the use of a protein described herein for the identification and/or validation of inhibitors of Hantavirus endonuclease and/or drugs against Hantavirus infections.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

### DESCRIPTION OF THE FIGURES

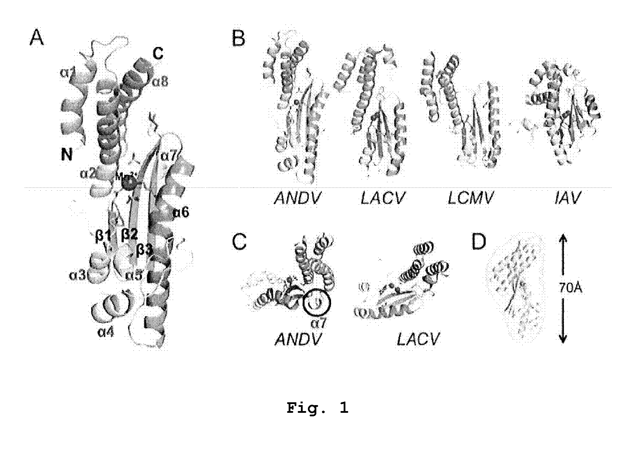

- **Figure 1**: shows the ADNV L¹⁻²⁰⁰ K127A structure and comparison with related cap-snatching endonucleases. A, Cartoon-representation of the ANDV L¹⁻²⁰⁰ K127A structure, secondary structure elements are labeled. The conserved structural core can be seen on the lower right side, the helix-bundle domain on the upper left side and additional structural elements on the lower left side. The manganese ion is shown as a sphere in the middle of the structure, N-and C-termini are marked. Side chains of the active site residues and bound sulfate/glycerol are shown as sticks. B, Comparison of ANDV L¹⁻²⁰⁰ K127A with the cap snatching endonucleases from LaCrosse Virus (LACV - 2XI7.pdb), Lymphocytic Choriomeningitis Virus (LCMV - 3JSB.pdb) and Influenza A Virus (IAV - 2W69.pdb). Structures are distributed and colored as in A. C, Top view of ANDV and LACV endonucleases to show the presence of the small helix α7 (encircled] in the ANDV structure. D, The SAXS structure (surface) matches the crystal structure of ANDV L¹⁻²⁰⁰ K127A (cartoon) and confirms its elongated and flat shape.

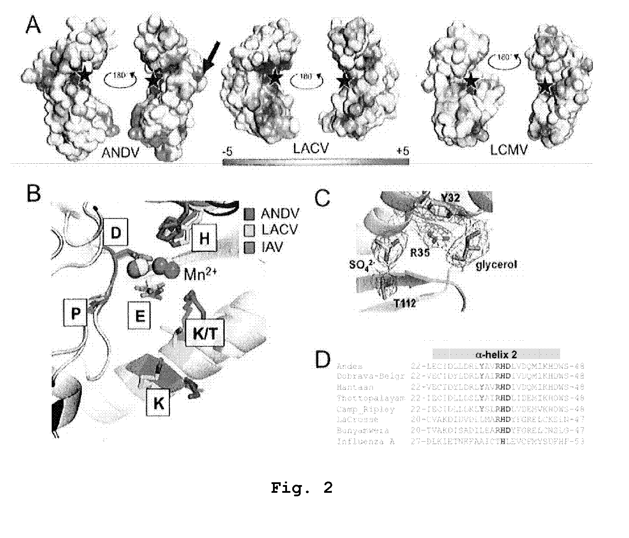

- **Figure 2**: shows the differences and similarities in the surface charge and active site of cap-snatching endonucleases. A, Electrostatic potential maps of the L protein endonucleases from ANDV, LACV and LCMV. The active site is marked with a black star, the location of helix cc7 in the ANDV structure with a black arrow. The ANDV surface has more positively charged patches than the other structures. B, Superposition of the active site residues from ANDV, LACV and IAV endonucleases shows the high similarities of side chains and Mn²⁺ ions. C, Electron density for a sulfate and a glycerol is present close to the active site of the ANDV endonuclease (2|Fo|-|Fc| map shown at 2σ); ligands and coordinating side chains shown as sticks). D, The arginine residue in helix α2, which coordinates the glycerol ligand in the ANDV L¹⁻²⁰⁰ structure is conserved in Bunyaviruses, but not present in IAV.

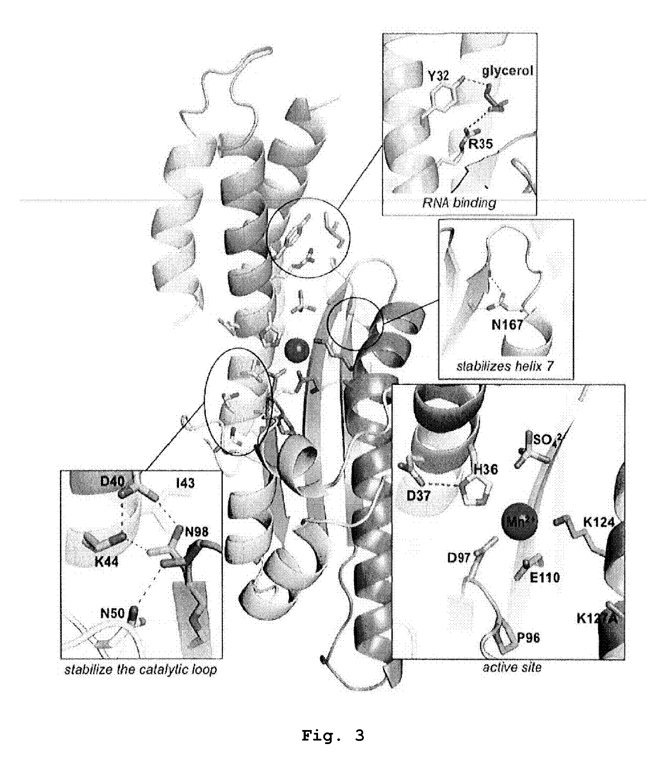

- **Figure 3**: shows that residues of ANDV endonuclease with an effect on the activity are grouped according to their role. Side chains of the 15 residues identified by Heinemann et al. (J Virol, 2013, 87(12):6975-85) are shown as sticks. Enlargements are shown, where the residues are grouped according to their role in the endonuclease. Important hydrogen bonds are highlighted with grey dashes, manganese ion as a sphere.

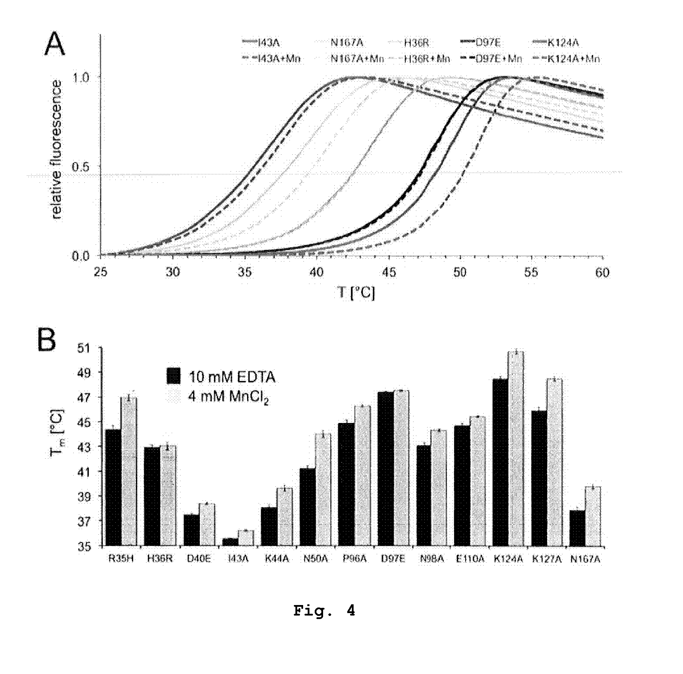

- **Figure 4**: shows the thermal stability of different ANDV L¹⁻²⁰⁰ mutants. A, thermal stability of five mutants using the Thermofluor assay is exemplarily shown, mutation of the metal coordinating side chains from His36 and Asp97 result in a loss of manganese-dependent stabilization. B, Melting temperatures (Tₘ) are shown for all purified mutants. All mutants were analyzed in the absence (black) and presence (grey) of 4 mM MnCl₂. The data presented are the average and standard deviations of four separate experiments.

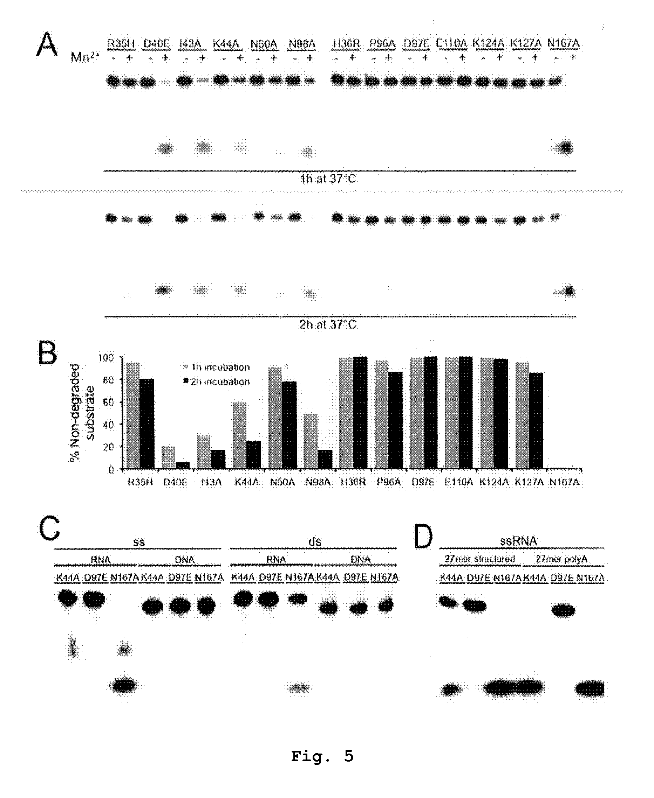

- **Figure 5**: shows the manganese dependent nuclease activity of different ANDV L¹⁻²⁰⁰ mutants. A, I µM of the indicated mutants was incubated with a radioactively labeled 27mer ssRNA in the presence and absence of 2mM MnCl₂. Reaction products were resolved on a denaturing gel after Ih and 2h at 37°C. B, Quantification of the gel shown in A. C, ANDV L¹⁻²⁰⁰ N167A prefers ssRNA over dsRNA as substrate and does not cleave DNA. D, unstructured polyA ssRNA is a better substrate for ANDV L¹⁻²⁰⁰ N167A than a ssRNA of equivalent length, but with intrinsic secondary structures.

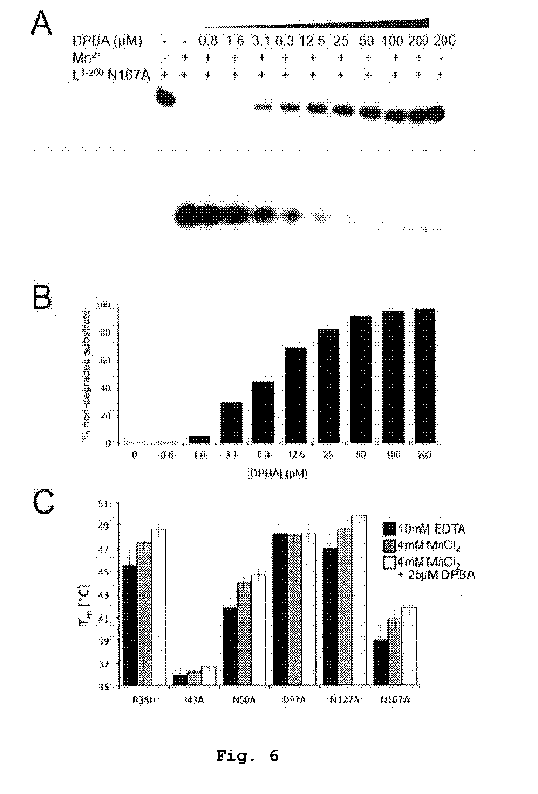

- **Figure 6**: shows that 2,4-dioxo-4-phenylbutanoic acid (DPBA) can inhibit and temperature stabilize ANDV L¹⁻²⁰⁰ N167A. A, I µM ANDV L¹⁻²⁰⁰ N167A was incubated with 2 mM MnCl₂ and increasing amounts of DPBA prior to the addition of the ssRNA substrate. Reaction products were resolved on a denaturing gel after incubation for Ih at 37°C. B, Quantification of the gel shown in A. C, Mutants of ANDV L¹⁻²⁰⁰ that were temperature stabilized by manganese ions were further temperature stabilized by addition of 25uM DPBA.

### EXAMPLES

### Example 1: Cloning, expression and purification of ANDV-L protein N-terminal domain

### Material and Methods

The cDNA of fragments encoding residues 1-163, 1-191, 1-194, 1-197, 1-200, 1-211, 1-214 and 1-228 from the L protein of ANDV (Chile 9717869 strain) were amplified by PCR and cloned into pOPIN-F (Berrow et al., Nucleic Acids Research, 2007, 35(6)), which introduces an N-terminal histidine-tag or pOPIN-M, which introduces an N-terminal MBP-protein and histidine-tag, both followed by a 3C protease cleavage site, to the cloned sequences. Single amino acid substitutions (Y32V, R35H, H36R, D37A, D40E, 143A, K44A, N50A, P96A, D97A, N98A, E110A, K124A, K127A and N167A) were generated in the backbone of pOPIN-F-L200wt by PCR site directed mutagenesis. All constructs were confirmed by DNA sequencing.

Proteins were expressed in *Escherichia coli* strain BL21 Gold (DE3) (Novagen) using LB medium at 17°C overnight after induction with 0.5 mM of 1 PTG. Cell pellets from harvested cultures were resuspended in 50 mM Tris-HCl pH 7.3, 300 mM NaCl, 10 mM imidazole, 10% glycerol, 10 mM MnCl₂ and 1 mM PMSF and disrupted by sonication. The soluble sample was loaded onto Ni-NTA affinity column, washed with 10 column volumes of lysis buffer with 50 mM imidazole and eluted with 5 volumes of lysis buffer containing 500 mM imidazole. The eluted protein was cleaved with GST-tagged 3C protease overnight at 4°C during dialysis against lysis buffer. The resulting untagged proteins were further purified by size exclusion chromatography using a Superdex 200 column with 50 mM Na-Citrate pH 5.5, 1 M NaCl and 5% glycerol. All proteins were concentrated, flash frozen and stored in aliquots at -20°C.

### Results

Expression of recombinant ANDV wild-type L protein in mammalian cells is severely impaired due to the enzymatic activity of its endonuclease domain (Heinemann et al., J Virol, 2013, 87(12):6975-85, Cheng et al., J Virol, 2014, 88(15):8706-12). We observed the same effect when we tried to produce the isolated endonuclease domain in bacterial cells. Expression plasmids for the first 163, 200 or 228 residues of ANDV L protein were successfully cloned in *E. coli* cloning strain DH5α both with an N-terminal His-tag or as MBP-fusion protein. Transformation in different expression strains was repeatedly unsuccessful for all constructs, assuming the leaky expression of only low amounts of the wild-type endonuclease is toxic to the cells. Heinemann et al. (J Virol, 2013, 87(12):6975-85) determined 15 different amino acid residues within the first 167 residues of ANDV L that seem to have an influence on the endonuclease activity, as their mutations yielded a detectable expression of L protein in mammalian cells. We cloned all 15 variants as N-terminally His-tagged constructs of 200 residues length (ANDV L¹⁻²⁰⁰) and tested them for expression in *E. coli* BL21 cells. The results are summarized in Table 1.

**Table 1. ADNV endonuclease mutants and their characteristics.**

| | *expression level in mammalian cells** | *expression level in bacteria* | *stability* | *stabilization by Mn*^{*2*+} | *nuclease activity* | *role* |
|---|---|---|---|---|---|---|
| **wt** | - | tox. | | | | |
| **H36R** | +++ | ++ | high | - | - | active site residues *essential for activity* |
| **D97E** | +++ | +++ | high | - | - | |
| **E110A** | +++ | ++ | high | + | - | |
| **P96A** | +++ | +++ | high | ++ | + | active site residues *not essential for activity* |
| **K124A** | +++ | ++ | high | ++ | + | |
| **K127A** | ++ | ++ | high | ++ | + | |
| **Y32V** | ++ | tox | | | | RNA binding |
| **R35H** | +++ | +++ | high | ++ | + | |
| **D37A** | + | tox | | | | stabilization of the active site |
| **D40E** | +++ | + | low | + | ++ | |
| **I43A** | +++ | + | low | + | ++ | |
| **K44A** | +++ | + | low | ++ | ++ | |
| **N50A** | + | +++ | med | ++ | + | |
| **N98A** | + | + | high | ++ | ++ | |
| **N167A** | + | + | low | ++ | +++ | stabilization of helix 7 |

| | | | | | | |
|---|---|---|---|---|---|---|
| ** in the context of the full-length protein* as *shown by Heinemann et al. (22)* tox: *toxic for E. coli cells* | | | | | | |

Two mutants (Y32V and D37A) behaved like the wild-type protein: no bacterial growth could be observed in *E. coli* BL21 cells. All other 13 mutants (R35H, H36R, D40E, I43A K44A, N50A, P96A, D97E, N98A, E110A, K124A, K127A and N167A) were successfully expressed with varying yields and purified via Nickel affinity purification followed by size exclusion chromatography. All mutants eluted from the Superdex 200 size exclusion column according to their expected size, assuming they all exist as monomers in solution. Noticeable slower growth kinetics of expressing *E*. *coli* cells were observed for mutant N167A.

### Example 2: Crystallization and Structure determination

### Material and Methods

ANDV L¹⁻²⁰⁰ K127A (10 mg/mL) was crystallized in the presence of 2 mM MnCl₂ in 100 mM NaAcetate pH 5.0, 27.5% PEG 3350 and 500mM AmS0₄ by sitting drop vapor diffusion. Crystals were flash frozen in liquid nitrogen with 8% Butanediol as cryoprotectant and diffraction data was collected to 2.4 Å at beamline ID29 at ESRF, Grenoble at a wavelength of 0.98 Å and 1.77 Å. Datasets were processed with iMosflm (Battye et al., Acta Crystallographica Section D-Biological Crystallography, 2011, 67:271-81). The structure was solved using the anomalous signal of the manganese ion bound to the protein with the AutoSol package implemented in PHENIX (Adams et al., Acta Crystallogr D Biol Crystallogr, 2010, 66(Pt 2):213-21) and refined by iterative cycles of manual model building in Coot (Emsley et al., Acta Crystallographica Section D-Biological Crystallography, 2010, 66:486-501) and computational refinement with PHENIX (data not shown).

Small Angle X-ray Scattering (SAXS) data for ANDV L¹⁻²⁰⁰ K127A in 50 mM Tris-HCl pH 7.3, 250 mM NaCl, 5% glycerol and 2 mM MnCl₂ were collected at the SAXS beamline P12 at the PETRA III storage ring (Deutsches Elektronen-Synchrotron, Hamburg) (Blanchet et al., J Appl Crystallogr, 2015, 48(Pt 2):431-43). Using a PILATUS 2M pixel detector at a sample-detector distance of 3.1 m and at an energy of 10 keV (λ = 1.24 Å), the range of momentum transfer 0.01 < s < 0.45 Å⁻¹ was covered (s = 4π sinθ/λ, where 20 is the scattering angle). 1, 2 and 5 mg/ml of protein were measured. SAXS data analysis was performed using the ATSAS 2.6 package. The forward scattering I(0) and the radius of gyration R_{g} were extracted from the Guinier approximation calculated with the AutoRG function within PRIMUS (Konarev et al., Journal of Applied Crystallography, 2003, 36:1277-82; Franke et al., Journal of Applied Crystallography, 2009, 42:342-6). GNOM provided the pair distribution function, P(r), of the particle and the maximum size Dₘₐₓ. *Ab initio* reconstructions were generated with the program DAMMIF. Ten independent DAMMIF runs were superimposed onto each other by SUPCOMB (Kozin and Svergun, Journal of Applied Crystallography, 2001, 34:33-41) and averaged using the program DAMAVER (Volkov and Svergun, Journal of Applied Crystallography, 2003, 36:860-4).

Structural figures were prepared with Pymol and Chimera (Pettersen et al., J Comput Chem, 2004, 25(13):1605-12). Electrostatic surfaces were calculated using PDB2PQR and APBS (Baker et al., Proc Natl Acad Sci USA, 2001, 98(18):10037-41; Dolinsky et al., Nucleic Acids Res, 2007, 35 (Web Server issue):W522-5).

### Results

### Structure determination of ANDV L¹⁻²⁰⁰ K127A

For structural studies we chose one of the very stable mutants that binds manganese (for the characterization of the mutants see below). Endonuclease constructs with less than 200 residues were expressed only in inclusion bodies and we tested different constructs with variable lengths (L¹⁻²⁰⁰ - L¹⁻²²⁸) in crystallization trials. We succeeded to crystallize ANDV L¹⁻²⁰⁰ K127A in the presence of Mn²⁺ after cleavage of the hexa-Histidine tag. The crystal structure was solved by single anomalous dispersion (SAD) in space group P42₁2 via phasing with the bound Mn-ion, allowing the building of a contiguous polypeptide chain for one molecule in the asymmetric unit and refinement to a resolution of 2.4 Å (Table 2). Electron density is visible for the full structure with only weak density for the loop between the first two α-helices (residues 13-20). The structure clearly shows the manganese ion bound to the active site (Figure 1A and 2B).

The crystal structure of ANDV L¹⁻²⁰⁰ K127A and its comparison to the cap-snatching endonucleases of the Bunyavirus LACV (Reguera et al., PLoS Pathog, 2010, 6(9):el001101), the Arenavirus LCMV (Dias et al., Nature, 2009, 458(7240):914-8; Morin et al., PLoS Pathog, 2010, 6(9):el001038) and the Orthomyxovirus IAV (Dias et al., ibid) is shown in Figure 1B. ANDV L¹⁻²⁰⁰ has an overall structure similar to the other endonucleases with the highest similarity to the closest relative LACV. Considering the almost lacking sequence homology between the four proteins, the structural homology is strikingly high. The central β-sheet with the long α-helix α5 that runs parallel to the β-sheet (Figure 1) forms the core of the protein and in all four endonucleases provides the important active site residues. All additional structural elements are helical, but the amount, the lengths as well as the position of the present α-helices vary significantly. In all structures the active site is located in a groove between two lobes. One lobe is in all structures formed by α helix bundle that consists of 2-3 α-helices from the N-terminus and at least one long α-helix from the C-terminal end of the domain (starting approximately with residue 160). ANDV has a short additional α-helix α7 between the last strand β3 and the C-terminal helix α8 (Figure 2C).

One of the obvious differences between the ANDV endonuclease and the other endonucleases are the overall dimensions of the domain: ANDV L¹⁻²⁰⁰ is very long and flat with approximate outer dimensions of 70Å x 45Å x 30Å, whereas the other structures more compact, the less they are related. Using small angle x-ray scattering it was demonstrated that this is not due to a crystallization artifact, but that the protein has the same outer dimensions in solution (Figure 1D).

### ANDV has a more positively charged surface than related endonucleases

As seen for the other cap-snatching endonucleases, the structurally more conserved active site of ANDV L¹⁻²⁰⁰ is located in a groove between two lobes. Most of the surface area of the domain, however, is formed by the large helical domain formed by the N- and C-terminal helices αI+2 and α7+8 (upper left in Figure 1) and three small helices α3-5 (lower left in Figure 1). These helices differ between the endonucleases not only in their conformation and orientation, but also in their amino acid composition. Figure 2A shows how this results in a significant variation in their surface charge distribution. The ANDV endonuclease has large positively charged patches surrounding the active site groove, which are not or only very weakly present in LACV and LCMV and to a large extent even replaced by negatively charged areas. It is known from previous studies (Heinemann et al., J Virol, 2013, 87(12):6975-85; Cheng et al., J Virol, 2014, 88(15):8706-12) that Hantaviruses possess a significantly more active endonuclease compared to other related viruses. The observed more positively charged protein surface surrounding the active site could increase the local concentration of the negatively charged nucleic acids and thereby cause a higher substrate binding affinity.

### Active site and ligand binding

A closer look to the active site of ANDV endonuclease in comparison with the LACV and IAV structures shows the close structural homology of the essential active site residues. The side chains of the conserved H-PD-D/E-K motif superimpose well as shown in Figure 2B. Also the manganese bound to the active site superimposes with the metal ion found in the other structures. It is therefore most probable that ANDV and other Hantaviruses possess the same two-metal dependent nuclease activity as LACV and IAV PA (Dias et al., Nature, 2009, 458(7240):914-8). Sequence comparisons suggested that the active site of the ANDV endonuclease is more closely related to that of IAV than LACV (Reguera et al., PLoS Pathog, 2010, 6(9):el001101; Heinemann et al., J Virol, 2013, 87(12):6975-85). Indeed Lys 124 of ANDV superimposes well with Lys 134 of IAV, whereas LACV has a threonine is this position.

Close to the active site electron density for a sulfate and for a glycerol is visible in the structure (Figure 2C). The glycerol ligand is coordinated by the conserved residues Tyr32 and Arg35 (Figure 2D), both of which were shown to have an influence on the endonuclease activity of the L protein (Heinemann et al., ibid).

### ANDV endonuclease mutants are distributed over the whole structure

With the crystal structure of the ANDV cap-snatching endonuclease at hand we have a way to propose a role for all 15 amino acid residues that were shown to have an influence on the expression phenotype of the L protein in mammalian cells (Heinemann et al., ibid) (Figure 3). We can group the residues in five different groups according to their role: (1) active site residues essential or (2) not essential for activity, residues involved in RNA binding (3) and residues stabilizing (4) the active site or (5) helix α7.

From sequence alignments the central catalytic residues were already proposed: His36, Pro96, Asp97 and Glu110 indeed form the core of the active site and the two lysines LysI24 and LysI27 (the latter being mutated to alanine in the present structure) emerge from helix α6 and are located to have an influence on the correct positioning of the nucleic acid substrate to the active site.

The other nine residues are not directly part of the endonuclease active site, but stabilize the tertiary structure or promote substrate binding. We grouped these residues in three subgroups: Asp40, Lys44, Asn50 and Asn98 form hydrogen bonds with each other and main chain atoms to position the loop that contains the PD motif of the active site. Ile43 forms hydrophobic interactions between the end of helix α2 and several hydrophobic side chains both from the β-sheet and from helix α7. Asp37 is positioning His36, but not involved in the catalytic reaction.

The second group consists of Tyr32 and Arg35, both are located in helix α2 and in the present structure binding to a glycerol solvent molecule. The binding to the hydroxyl groups of glycerol suggests that these residues are responsible for binding to the negatively charged RNA substrates.

The last group contains only one residue, AsnI67. It is located in the small helix α7, which is not present in the LACV endonuclease (see Figure ID) and stabilizes this helix via salt bridges with main chain atoms of strand β3.

### Example 3: In vitro endonuclease assays

### Material and Methods

The endonuclease activity was tested by incubation of 1 µM of ANDV-L200 mutant proteins with 0.1 µM of ³²P-labeled single or double stranded 27mer RNA or DNA substrate at 37°C for 60min in 50 mM Tris-HCl pH 7.3, 250 mM NaCl, 5% glycerol and 0.25 U/µl RNasin (Promega) in the absence or presence of 2 mM MnCl₂. The effect of DPBA on the nuclease activity was determined by adding increasing amounts to the mixture. Single stranded RNA or DNA radiolabeled substrates containing equivalent nucleotide sequences (5'-GA^{U}/_{T}GA^{U}/_{T}GC^{U}/_{T}A^{U}/_{T}CACCGCGC^{U}/_{T}CG^{U}/_{T}CG^{U}/_{T}C-3') were used to generate the double stranded substrates by hybridization with their corresponding nonlabeled complementary sequences. The reaction was stopped by adding loading buffer 2X (95% formamide, 18mM EDTA, 0.025% SDS, xylene cyanol and bromophenol blue) and heating the samples to 98°C for 5 min. The integrity of the labeled substrates was analyzed using denaturing 8M urea, 20% polyacrylamide TBE gel electrophoresis followed by phosphor screen autoradiography. Outcome images were digitized using a Typhoon scanner and the intensity of the bands was quantified by ImageJ software (Schneider et al., Nat Methods, 2012, 9(7):671-5).

The influence of Mn²⁺ ions and DPBA on protein thermal stability was measured by Thermofluor assays (Ericsson et al., Anal Biochem, 2006, 357(2):289-98) at a protein concentration of I0µM in I00mM Na-Phosphate pH 7, 300mM NaCl, 5% glycerol and either I0 mM EDTA, 4mM MnCl₂ or 25µM DPBA.

### Results

### Stabilization of ANDV mutants by Mn²⁺and endonuclease activity

We measured the thermal stability of the purified mutants by a Thermofluor assay (Ericson et al, Anal Biochem, 2006, 357(2):289-98) in analogy to previously described experiments with the IAV and LACV cap-snatching endonucleases (Dias et al., Nature, 2009, 458(7240):914-8; Reguera et al., PLoS Pathog, 2010, 6(9):el001101). We found that the L¹⁻²⁰⁰ constructs are most stable at low pHs, as expected from their calculated pl of approx. 7.5 and at high salt concentrations (data not shown).

Therefore we used pH 5.5 and 1 M NaCl for the purification and crystallization experiments. For the analysis of the mutants, SAXS experiments and activity assays we chose a more physiological buffer with pH 7.3 and 250 mM NaCl. Stability of the mutants varies with an apparent melting temperature of 35.5 °C for the least stable L¹⁻²⁰⁰ I43A and 48.5 °C for the most stable L¹⁻²⁰⁰ K124A (Figure 4 and Table 1). For most mutants the thermal stability is enhanced by approx. 2°C in the presence of manganese, presumably due to metal binding (Figure 4B). In contrast, mutations in the active site residues directly involved in metal binding (His36, Asp97, GlullO) abolish this stabilizing effect.

We also performed endonuclease assays to compare the amount of enzymatic activity still present in the different mutants. We used a 5'-radioactively labeled 27mer ssRNA molecule with a mixed sequence as substrate, resolved the reaction products on a denaturing gel and quantified the bands by phosphor-imaging (Figure 5A). The level of endonucleolytic activity varies for the different mutants, but is always dependent on manganese: no RNA degradation could be observed in the presence of other divalent metal ions (data not shown).

As expected, active site mutants H36R, D97E and E110A are inactive. A very low activity can be observed for P96A, K124A and K127A. All three side chains are part of the active site, but not directly involved in metal binding and therefore obviously not essential, but very important for activity. Also Arg35, the residue we propose to bind the RNA substrate, and Asn50, which stabilizes the active site loop via salt bridges with main chain atoms, have a strong influence on the endonuclease activity. The next group of mutants, D40A, I43A, K44A and N89A possess an intermediate activity. These residues all belong to the group that stabilizes the structure and therefore have an indirect influence on the active center. Finally the most active mutant, N167A, is also one that could not be grouped with any of the other mutants. AsnI67 is located in the small helix α7 that is not present in the LACV endonuclease, stabilizing it via salt bridges with main chain atoms of β3.

### ANDV cap-snatching endonuclease prefers ssRNA over dsRNA and does not cleave DNA

To investigate substrate specificity of the ANDV cap-snatching endonuclease we performed the activity assays using the most active mutant N167A and K44A and different substrates: a 27mer ssRNA that can form secondary structures, the equivalent dsRNA and the analog ssDNA and dsDNA molecules. ANDV L¹⁻²⁰⁰ N167A and K44A clearly prefer ssRNA over dsRNA, but can also cleave dsRNA. Both ssDNA and dsDNA were not degraded (Figure 5C). Additionally, we compared the activity on ssRNA with different sequences: a polyA 27mer is preferred over the 27mer with heterogeneous sequence, which can form a hairpin-like structure (Figure 5D). In general, longer RNAs are degraded much faster by the endonuclease than shorter constructs, presumably due to a higher binding affinity (data not shown).

### ANDV L¹⁻²⁰⁰ N167A is inhibited by DPBA

The metal dependent cap-snatching endonuclease is an attractive target in the development of antiviral drugs against infection with segmented negative strand RNA viruses. Several potential substances were described for influenza (Hsu et al., Antimicrob Agents Chemother, 2012, 56(2):647-57; Kowalinski et al., PLoS Pathog. 2012, 8(8):el002831; Pala et al., ACS Med Cheni Lett, 2015, 6(8):866-71; Shibagaki et al., J Virol Methods, 2014, 202:8-14), but screening of possible compounds is challenging for Hantaviruses due to the high activity of its cap-snatching endonuclease and the resulting difficulties in recombinant protein production. We identified mutants that can be produced in high quantities in *E. coli* and still exhibit endonuclease activity. To proof suitability for validation of potential inhibitors, we incubated the ANDV L¹⁻²⁰⁰ R35H, 143A, N50A, D97A, N127A and N167A constructs with Mn²⁺ and the known influenza virus endonuclease inhibitor DPBA (2,4-dioxo-4-phenylbutanoic acid) and measured thermal stability as described above. All mutants that show higher melting temperatures upon addition of manganese show an additional increase of the melting temperature upon further addition of DPBA (Figure 6A). The active site mutant D97A, which is not stabilized by manganese, is also not affected by DPBA.

To proof that DPBA not only binds to the active site of ANDV L¹⁻²⁰⁰, but also inhibits its endonuclease activity, we incubated the most active construct ANDV L¹⁻²⁰⁰ N167A with Mn²⁺ and DPBA prior to the addition of the RNA substrate. Figure 6B shows a clear inhibition of the endonuclease activity in a range comparable for IAV and LACV (Dias et al., Nature, 2009, 458(7240):914-8; Reguera et al., PLoS Pathog, 2010, 6(9): el001101).

## Claims

1. Method for the identification and/or validation of inhibitors of Hantavirus endonuclease and/or drugs for treating or preventing Hantavirus infections, comprising
(a) contacting a protein comprising an attenuated Hantavirus endonuclease or a fragment thereof having endonuclease activity with a candidate compound;
(b) determining the endonuclease activity of the protein; and
(c) comparing the endonuclease activity of step b) with the endonuclease activity of the protein in the absence of the candidate compound;
wherein a decreased endonuclease activity of the protein in the presence of the candidate compound indicates that the candidate compound is an inhibitor of Hantavirus endonuclease and/or is suitable for treating or preventing Hantavirus infections.

2. Method according to claim 1, wherein the attenuated Hantavirus endonuclease or fragment thereof comprises a mutation in relation to the wildtype Hantavirus endonuclease, preferably in an amino acid involved in the stabilization of the tertiary structure of the Hantavirus endonuclease.

3. Method according to claim 1, wherein the attenuated Hantavirus endonuclease or fragment thereof comprises a mutation outside the active site of the endonuclease in relation to the wildtype Hantavirus endonuclease.

4. The method according to any of claims 1-3, wherein the endonuclease or fragment thereof is a recombinantly expressed protein, preferably a protein expressed in a bacterial host cell such as *Escherichia coli.*

5. The method according to any of claims 1-4, wherein the endonuclease or fragment thereof comprises a mutation in one or more positions corresponding to N167, R35, D40, I43, K44, N50, P96, N98, K124, and K127 of the Andes virus L protein shown in SEQ ID NO:1, and preferably positions corresponding to N167, D40, I43, K44, N50, and N98 of the Andes virus L protein shown in SEQ ID NO:1.

6. The method according to claim 5, wherein the endonuclease or fragment thereof comprises a mutation in the position corresponding to N167 of the Andes virus L protein shown in SEQ ID NO:1.

7. The method according to claim 6, wherein the endonuclease or fragment thereof comprises an alanine at the position corresponding to N167 of the Andes virus L protein shown in SEQ ID NO:1.

8. The method according to any of claims 1-7, wherein steps a) and b) are performed in the presence of manganese, preferably manganese chloride.

9. The method according to claim 8, wherein manganese chloride is present in a concentration from 0.1 to 10 mM, preferably 0.5 to 5 mM, more preferably 1 to 3 mM.

10. The method according to any of claims 1-9, wherein the endonuclease activity of the protein is determined in step b) by incubating the endonuclease or fragment thereof with a labeled DNA or RNA substrate and subsequently determining the integrity of the substrate.

11. The method according to any of claims 1-10, wherein the Hantavirus endonuclease is an endonuclease from Andes virus, Amur virus, Asama virus, Azagny virus, Bayou virus, Black Creek Canal virus, Bloodland Lake virus, Blue River virus, Cano Delgadito virus, Calabazo virus, Carrizal virus, Catacamas virus, Choclo virus, Dobrava-Belgrade virus, El Moro Canyon virus, Gou virus, Hantaan River virus, Huitzilac virus, Imjin virus, Isla Vista virus, Khabarovsk virus, Laguna Negra virus, Limestone Canyon virus, Magboi virus, Maripa virus, Monongahela virus, Montano virus, Mouyassue virus, Muleshoe virus, Muju virus, New York virus, Nova virus, Oran virus, Oxbow virus, Playa de Oro virus, Prospect Hill virus, Puumala virus, Rockport virus, Rio Mamore virus, Rio Segundo virus, Sangassou virus, Saaremaa virus, Seoul virus, Serang virus, Sin Nombre virus, Soochong virus, Tanganya virus, Thailand virus, Thottapalayam virus, Topografov virus, Tula virus, or Xuan Son virus.

12. The method according to any of claims 1-11, wherein the Hantavirus endonuclease is an endonuclease from *Andes virus* (ANDV).

13. Recombinant protein comprising an enzymatically active Hantavirus endonuclease or fragment thereof, wherein the endonuclease or fragment thereof comprises a mutation in one or more sequence positions selected from the group consisting of positions corresponding to N167, R35, D40, I43, K44, N50, P96, N98, K124, and K127 of the Andes virus L protein shown in SEQ ID NO:1, preferably positions corresponding to N167, D40, I43, K44, N50, and N98 of the Andes virus L protein shown in SEQ ID NO:1, most preferably position corresponding to N167 of the Andes virus L protein shown in SEQ ID NO:1.

14. Host cell expressing a protein according to claim 14.

15. Use of a protein according to claim 13 or a host cell according to claim 14 for the identification and/or validation of inhibitors of Hantavirus endonuclease and/or drugs against Hantavirus infections.
